(19) Europäisches Patentamt
European Patent Office
Office européen des brevets

(11) EP 1 255 757 B1

(12) # FASCICULE DE BREVET EUROPEEN

(45) Date de publication et mention de la délivrance du brevet:
**18.06.2003 Bulletin 2003/25**

(21) Numéro de dépôt: **01907822.9**

(22) Date de dépôt: **16.02.2001**

(51) Int Cl.7: **C07D 471/04**

(86) Numéro de dépôt international:
**PCT/FR01/00463**

(87) Numéro de publication internationale:
**WO 01/060822 (23.08.2001 Gazette 2001/34)**

(54) **DERIVES D'AMINOACIDES ET LEURS UTILISATION EN TANT QU'INHIBITEURS DE LA NEP, L' ACE ET L' ECE**

AMINOSÄUREDERIVATE UND DEREN VERWENDUNG ALS INHIBITOREN VON NEP, ACE UND ECE

AMINO ACID DERIVATIVES AND USE THEREOF AS NEP, ACE AND ECE INHIBITORS

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **17.02.2000 FR 0001937**

(43) Date de publication de la demande:
**13.11.2002 Bulletin 2002/46**

(73) Titulaires:
- **INSTITUT NATIONAL DE LA SANTE ET DE LA RECHERCHE MEDICALE (INSERM)**
  **75654 Paris Cédex 13 (FR)**
- **LES LABORATOIRES SERVIER**
  **92415 Courbevoie Cédex (FR)**

(72) Inventeurs:
- **ROQUES, Bernard, P.**
  **F-75014 Paris (FR)**
- **FOURNIE-ZALUSKI, Marie-Claude**
  **F-75011 Paris (FR)**
- **INGUIMBERT, Nicolas**
  **F-94230 Cachan (FR)**
- **PORAS, Hervé**
  **F-94140 Alfortville (FR)**
- **SCALBERT, Elizabeth**
  **F-75016 Paris (FR)**
- **BENNEJEAN, Caroline**
  **F-94220 Charenton Le Pont (FR)**
- **RENARD, Pierre**
  **F-78150 Le Chesnay (FR)**

(56) Documents cités:
**EP-A- 0 449 523**
**WO-A-97/24341**
**WO-A-97/32874**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 255 757 B1

## Description

La présente invention concerne de nouveaux dérivés d'aminoacides N-mercaptoacylés, leur procédé de préparation et les compositions pharmaceutiques qui les contiennent.

De nombreuses demandes de brevets décrivent des dérivés d'acides aminés utiles en tant qu'inhibiteurs de l'endopeptidase neutre (NEP) (EP449523), en tant qu'inhibiteurs de l'enzyme de conversion de l'endothéline (ECE) (WO9732874), ou encore en tant qu'inhibiteurs mixtes de la NEP et de l'enzyme de conversion de l'angiotensine I (ACE). Le rôle pharmacologique joué par ces enzymes est :

- pour l'ACE, de transformer l'angiotensine I en angiotensine II et dégrader la bradykinine en peptides inactifs,
- pour la NEP, de dégrader la bradykinine et le peptide atrial natriurétique en peptides inactifs,
- pour l'ECE, de transformer la big endothéline-1 en endothéline-1.

L'angiotensine II, l'endothéline, la bradykinine et le peptide atrial natriurétique sont les peptides les plus importants à ce jour impliqués dans la régulation du tonus vasculaire, du remodelage cardiovasculaire et de l'homéostasie hydroélectrolytique. Leur métabolisme est essentiellement contrôlé par ces trois enzymes. L'inhibition de l'une et/ou de l'autre de ces enzymes permet de restaurer une balance peptidergique optimale en favorisant les peptides vasodilatateurs, antitrophiques et natriurétiques (bradykinine, peptide atrial natriurétique) aux dépens des peptides vasoconstricteurs, trophiques et antinatriurétiques (angiotensine II, endothéline-1) d'où un bénéfice thérapeutique cardiovasculaire.
Les propriétés pharmacologiques des inhibiteurs mixtes ACE/NEP décrits dans l'art antérieur négligent le rôle cardiovasculaire majeur du système endothéline (Haynes W. G. et col., Journal of Hypertension, 1998, 16 (8), pp. 1081-1098) ainsi que l'implication mise en évidence de la NEP dans la dégradation de l'endothéline-1 (Vijayaraghavan J. et col., J. Biol. Chem., 1990, 265, pp. 14150-14155). Ainsi, le traitement par des inhibiteurs mixtes ACE/NEP a pour conséquence l'augmentation des taux d'endothéline-1 qui, à long terme, peut se révéler néfaste au bénéfice thérapeutique attendu. Ce problème trouve sa solution dans l'obtention, au sein d'une même molécule des trois types d'inhibition, permettant de contre-réguler cette activation et amenant ainsi à une efficacité thérapeutique renforcée et maintenue. Ainsi la mise au point de molécules inhibant ces trois enzymes représente une avancée très importante dans le traitement de l'hypertension artérielle et des maladies cardiovasculaires.

Les composés de la présente invention sont nouveaux et sont d'excellents agents inhibiteurs triples, c'est-à-dire qu'ils sont capables d'inhiber à la fois la NEP, l'ACE et l'ECE.

La présente invention concerne plus particulièrement les composés de formule (I) :

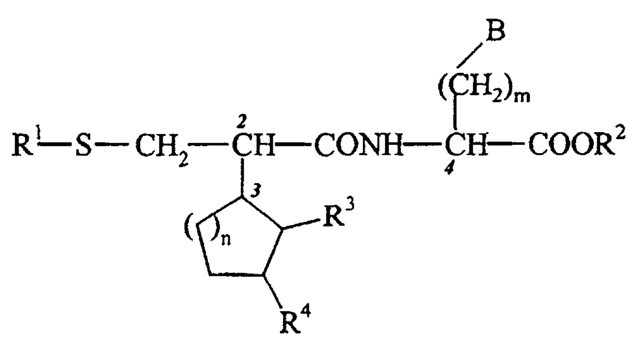

(I)

dans laquelle :

- n représente un nombre entier tel que $0 \leq n \leq 3$,
- m représente un nombre entier tel que $0 \leq m \leq 6$,
- $R^3$ et $R^4$ forment ensemble, avec les deux atomes de carbone qui les portent un groupement phényle non substitué ou substitué par 1 à 3 groupements, identiques ou différents, choisis parmi alkyle, alkényle, alkynyle, alkoxy, hydroxy, alkylthio, mercapto, cyano, nitro, amino, alkylamino, dialkylamino, polyhalogénoalkyle, azido, carboxy, alkoxycarbonyle, amido, carbamoyle, formyle, acyle, aryle, hétéroaryle ou atomes d'halogène,
- B représente un groupement hétéroaryle,
- $R^2$ représente un atome d'hydrogène ou un groupement alkyle, alkényle, cycloalkyle, cycloalkylalkyle, acyle,

aryle, arylalkyle ou aroyle,

➢ $R^1$ représente un atome d'hydrogène, un groupement acyle, aroyle ou cycloalkyl-carbonyle ou un groupement de formule (II) :

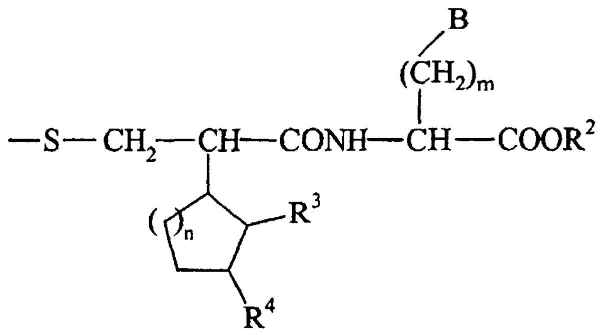

(II)

dans laquelle m, n, $R^2$, $R^3$, $R^4$ et B sont tels que définis précédemment,

étant entendu que :

- par « alkyle » on entend un groupement alkyle à chaîne linéaire ou ramifiée contenant 1 à 6 atomes de carbone,
- par « alkényle » on entend un groupement alkyle contenant 2 à 6 atomes de carbone et une ou plusieurs double-liaisons,
- par « alkynyle » on entend un groupement alkyle contenant 2 à 6 atomes de carbone et une ou plusieurs triple-liaisons,
- par « cycloalkyle » on entend un groupement alkyle cyclique contenant 3 à 8 atomes de carbone,
- par « acyle » on entend un groupement RCO dans lequel R représente un groupement alkyle tel que défini précédemment,

  les groupements « alkyle », « alkényle » et « alkynyle » pouvant être substitués par un ou plusieurs groupements, identiques ou différents, choisis parmi hydroxy, alkoxy, polyhalogénoalkyle, amino ou atomes d'halogène,

  et les groupements « cycloalkyle » et « cycloalkylalkyle » pouvant être substitués sur la partie cyclique par un ou plusieurs groupements, identiques ou différents, choisis parmi hydroxy, alkoxy, polyhalogénoalkyle, amino ou atomes d'halogène,
- par « aryle », on entend un groupement phényle ou naphtyle non substitué ou substitué par ou plusieurs groupements, identiques ou différents, choisis parmi alkyle, alkényle, alkynyle, alkoxy, hydroxy, alkylthio, mercapto, cyano, nitro, amino, alkylamino, dialkylamino, polyhalogénoalkyle, azido, carboxy, alkoxycarbonyle, amido, carbamoyle, formyle, acyle ou atomes d'halogène,
- par « hétéroaryle » on entend tout groupement aromatique mono ou polycyclique contenant 1 à 3 hétéroatomes choisis parmi oxygène, soufre et azote, ces groupements étant non substitués ou substitués par un ou plusieurs groupements, identiques ou différents, choisis parmi alkyle, alkényle, alkynyle, alkoxy, hydroxy, alkylthio, mercapto, cyano, nitro, amino, alkylamino, dialkylamino, polyhalogénoalkyle, azido, carboxy, alkoxycarbonyle, amido, carbamoyle, formyle, acyle ou atomes d'halogène, les groupements polycycliques pouvant être par ailleurs partiellement ou totalement hydrogénés sur un des cycles,

  leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels $R^1$ représente un atome d'hydrogène ou un groupement acyle.

La valeur préférée pour m et n est 1.

Les groupements $R^2$ préférés sont l'atome d'hydrogène et les groupements alkyle et arylalkyle.

De façon avantageuse, l'invention concerne les composés de formule (I) pour lesquels $R^3$ et $R^4$ forment ensemble, avec les deux atomes de carbone qui les portent un groupement phényle substitué.

Plus avantageusement, l'invention concerne les composés de formule (I) pour lesquels $R^3$ et $R^4$ forment ensemble, avec les deux atomes de carbone qui les portent un groupement phényle substitué par un atome d'halogène et plus particulièrement par un atome de brome ou substitué par un groupement alkoxy ou alkylthio et plus particulièrement les groupements méthoxy et méthylthio.

De façon encore plus avantageuse, l'invention concerne les composés de formule (I) substitués en *(2)* par un groupement indane substitué en position 5 par un atome d'halogène et plus particulièrement un atome de brome ou par un groupement alkoxy et plus particulièrement un groupement méthoxy.

Les groupements B préférés sont les hétéroaryles contenant un groupement NH comme par exemple les groupements indolyle, imidazolyle, pyrrolopyridinyle, pyrroloquinolinyle, pyrrolyle, pyrrolopyrazinyle et plus particulièrement les groupements indolyle, 1*H*-pyrrolo[2,3-*b*]pyridine et 1*H*-pyrrolo[3,2-*h*]quinolinyle.

La configuration préférée des composés de formule (I) est 2S-3R, et plus particulièrement 2S-3R-4S.

Encore plus avantageusement l'invention concerne le :

* *N*-[2-(5-bromo-2,3-dihydro-1*H*-indèn-1-yl)-3-mercaptopropanoyl]tryptophane,
* *N*-[2-(5-bromo-2,3-dihydro-1*H*-indèn-1-yl)-3-mercaptopropanoyl]tryptophane (2S-3R-4S),
* *N*-[2-(5-chloro-2,3-dihydro-1*H*-indèn-1-yl)-3-mercaptopropanoyl]tryptophane,
* *N*-[(2,3-dihydro-1*H*-indèn-1-yl)-3-mercaptopropanoyl]tryptophane,
* *N*-[3-mercapto-2-(1,2,3,4-tétrahydro-1-naphtalényl)propanoyl]tryptophane,
* *N*-[2-(5-méthoxy-2,3-dihydro-1*H*-indèn-1-yl)-3-mercaptopropanoyl]tryptophane,
* *N*-[2-(4-méthoxy-2,3-dihydro-1*H*-indèn-1-yl)-3-mercaptopropanoyl]tryptophane,
* *N*-[2-(5-éthoxy-2,3-dihydro-1*H*-indèn-1-yl)-3-mercaptopropanoyl]tryptophane,
* *N*-[2-(5-hydroxy-2,3-dihydro-1*H*-indèn-1-yl)-3-mercaptopropanoyl]tryptophane,
* *N*-{2-[5-(méthylthio)-2,3-dihydro-1*H*-indèn-1-yl]-3-mercaptopropanoyl}tryptophane,
* *N*-[2-(5-cyano-2,3-dihydro-1*H*-indèn-1-yl)-3-mercaptopropanoyl]tryptophane,
* *N*-[2-(5-bromo-2,3-dihydro-1*H*-indèn-1-yl)-3-mercaptopropanoyl]-3-(1*H*-pyrrolo [2,3-*b*]pyridin-3-yl)alanine,
* *N*-[2-(5-bromo-2,3-dihydro-1*H*-indèn-1-yl)-3-mercaptopropanoyl]-3-(1*H*-pyrrolo [2,3-b]pyridin-3-yl)alanine (2S-3R-4S),
* *N*-[2-(5-bromo-2,3-dihydro-1*H*-indèn-1-yl)-3-mercaptopropanoyl]-1-méthyl-tryptophane,
* 3-(1-benzothiophèn-3-yl)-*N*-[2-(5-bromo-2,3-dihydro-1*H*-indèn-1-yl)-3-mercaptopropanoyl]alanine,
* *N*-[2-(5-bromo-2,3-dihydro-1*H*-indèn-1-yl)-3-mercaptopropanoyl]-3-(3-pyridinyl) alanine,
* *N*-[2-(5-bromo-2,3-dihydro-1*H*-indèn-1-yl)-3-mercaptopropanoyl]-3-(2-quinolinyl) alanine,
* *N*-[2-(5-bromo-2,3-dihydro-1*H*-indèn-1-yl)-3-mercaptopropanoyl]-5-méthoxytryptophane (2S-3R-4S),
* *N*-[2-(5-bromo-2,3-dihydro-1*H*-indèn-1-yl)-3-mercaptopropanoyl]-5-fluorotryptophane (2S-3R-4S),
* *N*-[2-(5-bromo-2,3-dihydro-1*H*-indèn-1-yl)-3-mercaptopropanoyl)-3-(1*H*-pyrrolo[3,2-*h*] quinolin-3-yl)alanine (2S-3R-4S).

La présente invention concerne également le procédé de préparation des composés de formule (I) caractérisé en ce que l'on utilise comme produit de départ le composé de formule (III) :

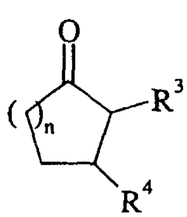

(III)

dans laquelle $R^3$, $R^4$ et n sont tels que définis dans la formule (I),

que l'on soumet à un agent réducteur comme $NaBH_4$ par exemple pour obtenir le composé de formule (IV) :

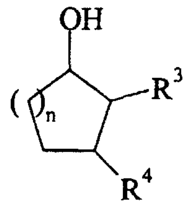

(IV)

dans laquelle n, $R^3$ et $R^4$ sont définis comme précédemment,
que l'on transforme avec un agent d'halogénation comme $Me_3SiBr$ par exemple en dérivé halogéné correspondant de formule (V) :

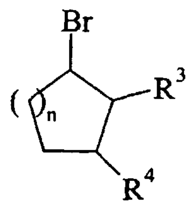

(V)

dans laquelle $R^3$, $R^4$ et n sont tels que définis précédemment,
sur lequel on condense, en milieu basique le 2-(diéthoxyphosphoryl)acétate d'éthyle pour conduire au composé de formule (VI) :

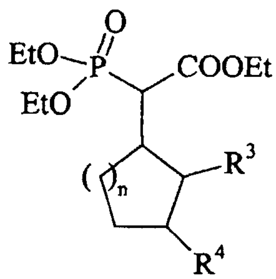

(VI)

dans laquelle $R^3$, $R^4$ et n sont définis comme précédemment,
sur lequel on fait réagir du formaldéhyde en milieu basique pour obtenir le composé de formule (VII) :

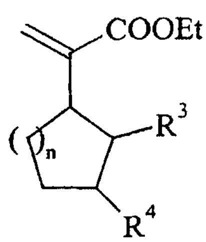

(VII)

dans laquelle $R^3$, $R^4$ et n sont définis comme précédemment,
qui est saponifié en présence de soude pour conduire au composé de formule (VIII) :

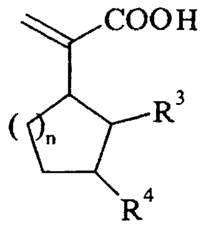

(VIII)

dans laquelle $R^3$, $R^4$ et n sont définis comme précédemment,
sur lequel on condense un composé de formule (IX) :

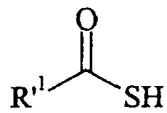

(IX)

dans laquelle $R'^1$ représente un groupement alkyle, aryle ou cycloalkyle,
pour obtenir le composé de formule (X) :

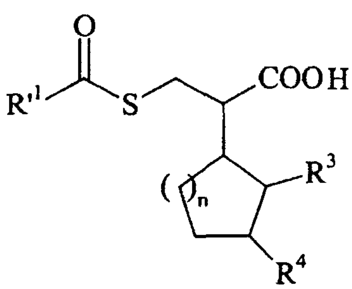

(X)

dans laquelle $R^3$, $R^4$, $R'^1$ et n sont tels que définis précédemment,
sur lequel on condense, en présence d'un agent de couplage comme le 1-(3-diméthylaminopropyl)-3-éthylcarbodiimide par exemple, un composé de formule (XI) :

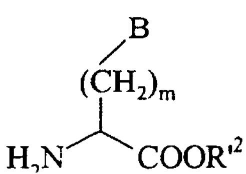

(XI)

dans laquelle B et m sont tels que définis dans la formule (I), et $R'^2$ représente un groupement alkyle, alkényle, cycloalkyle, cycloalkylalkyle, aryle, acyle, arylakyle ou aroyle,
pour conduire au composé de formule (I/a), cas particulier des composés de formule (I):

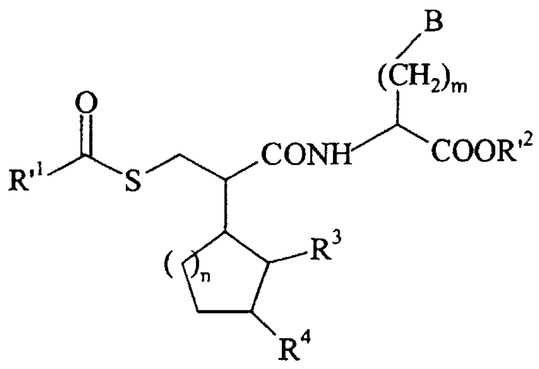

(I/a)

dans laquelle R'$^1$, $R^3$, $R^4$, R'$^2$, m, n et B sont définis de la même façon que précédemment, qui peut être partiellement ou totalement saponifié en milieu basique pour conduire au composé de formule (I/b), cas particulier des composés de formule (I) :

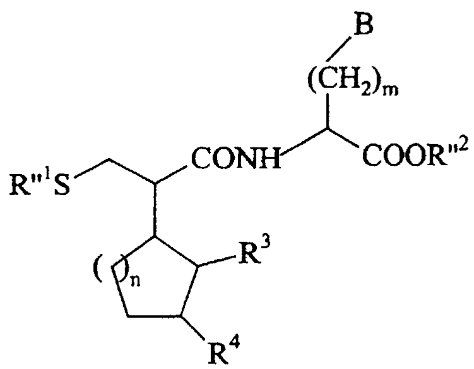

(I/b)

dans laquelle $R^3$, $R^4$, m, n et B sont tels que définis précédemment, R"$^1$ représente un groupement R'$^1$ ou un atome d'hydrogène, et R"$^2$ représente un groupement R'$^2$ ou un atome d'hydrogène, étant entendu que l'un au moins des groupements R"$^1$ et R"$^2$ représente un atome d'hydrogène,
composé de formule (I/b) qui peut être placé, lorsque R"$^1$ représente un atome d'hydrogène, en milieu oxydant pour obtenir le composé de formule (I/c), cas particulier des composés de formule (I):

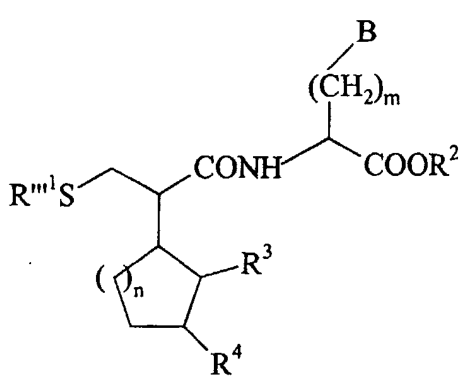

(I/c)

dans laquelle $R^2$, $R^3$, $R^4$, m, n et B sont tels que définis précédemment, et R"'$^1$ représente un groupement de formule (II),
les composés de formule (I/a) à (I/c) formant la totalité des composés de l'invention, et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

La présente invention concerne également le procédé de préparation des composés de formule (I) caractérisé en ce que l'on utilise comme produit de départ le composé de formule (X) tel que défini précédemment dont on sépare par chromatographie les diastéréoisomères, pour conduire aux composés de formule (Xa) et (Xb) :

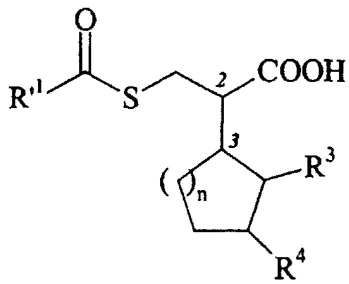

(2R 3R, 2S 3S) (Xa)

(2R 3S, 2S 3R) (Xb)

dans laquelle $R'^1$, $R^3$, $R^4$ et n sont définis comme précédemment, composé de formule (Xb) dont on peut former un sel avec une amine chirale comme la (R)-(+)-α-méthylbenzylamine par exemple, pour conduire, après dédoublement par recristallisations successives au composé de formule (Xb') :

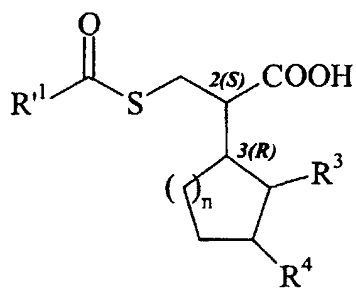

(2S, 3R) (Xb')

dans laquelle $R'^1$, $R^3$, $R^4$ et n sont tels que définis précédemment, sur lequel on condense, en présence d'un agent de couplage comme le EDCI, un composé de formule (XIa) :

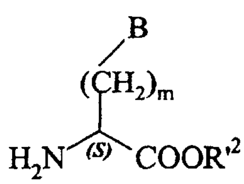

(XIa)

dans laquelle $R'^2$, m et B sont tels que définis précédemment, pour obtenir le composé de formule (I/a'), cas particulier des composés de formule (I/a) :

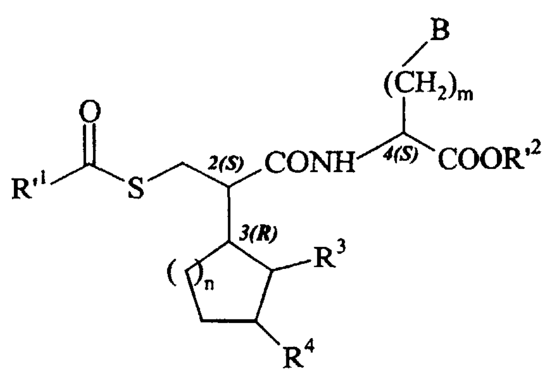

(I/a')

dans laquelle $R'^1$, $R'^2$, $R^3$, $R^4$, m, n et B sont définis de la même façon que précédemment,
les diastéréoisomères (2R, 3S), (2R, 3R) et (2S, 3R) étant obtenus de façon analogue à partir des composés (Xa) ou (Xb) correspondants,
ces composés pouvant par ailleurs être obtenus par condensation du composé de formule (XIa) sur le composé de formule (Xa) ou (Xb) suivie d'une séparation chromatographique.

Les composés de formule (III) sont soit commerciaux soit aisément accessibles à l'homme du métier par des réactions chimiques classiques.

Les composés de la présente invention possèdent des propriétés pharmacologiques très intéressantes puisqu'ils permettent une inhibition simultanée :

- de l'enzyme de conversion de l'angiotensine I (ACE), responsable de la transformation de l'angiotensine I en angiotensine II et de la dégradation de la bradykinine en peptides inactifs,
- de l'endopeptidase neutre (NEP) responsable de la dégradation de la bradykinine et du peptide atrial natriurétique en peptides inactifs,
- et de l'enzyme de conversion de l'endothéline (ECE) responsable de la transformation de la big endothéline-1 en endothéline-1.

Ces enzymes jouent un rôle déterminant dans l'établissement des proportions entre peptides vasodilatateurs, antitrophiques et natriurétiques d'une part (bradykinine, peptide atrial natriurétique) et peptides vasoconstricteurs, trophiques et antinatriurétiques d'autre part (angiotensine II, endothéline 1).
Il a par ailleurs été récemment montré que l'endopeptidase neutre était impliquée dans les mécanismes de dégradation de l'endothéline-1. L'inhibition de l'une et/ou l'autre de ces enzymes permet de moduler cette balance peptidergique.

Ainsi, les nombreux inhibiteurs mixtes ACE/NEP décrits dans la littérature augmentent la proportion en peptides vasodilatateurs aux dépens des peptides vasoconstricteurs. Néanmoins, cette approche néglige le rôle joué par le système endothéline, ce qui est d'autant plus délétère que ces inhibiteurs mixtes, de par l'inhibition de la NEP augmentent les taux d'endothéline-1, ce qui se traduit par une réduction du bénéfice thérapeutique attendu.

L'inhibition triple évite l'accumulation d'endothéline-1. De ce fait, elle aboutit à une efficacité thérapeutique renforcée et maintenue, ainsi qu'à un élargissement du spectre d'action des composés.

Ces propriétés justifient leur application en thérapeutique pour traiter les hypertensions artérielles y compris les hypertensions artérielles pulmonaires, l'ischémie myocardique, l'angine de poitrine, les insuffisances cardiaques, les vasculopathies comprenant les vasculopathies diabétiques, l'athérosclérose et la resténose post-angioplastie, les insuffisances rénales aiguës ou chroniques, les maladies cérébrovasculaires comprenant le stroke et les hémorragies sous-arachnoïdiennes, les ischémies périphériques, ainsi que la toxicité à la cyclosporine.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures en 1 ou plusieurs prises.

Les exemples suivants illustrent l'invention et ne la limitent en aucune façon. Les préparations suivantes conduisent à des composés de l'invention ou à des intermédiaires de synthèse utiles dans la préparation de l'invention.

**<u>Préparation 1</u> : 5-Bromo-1-indanone**

*<u>Stade A</u> : 1-(4-Bromophényl)-3-chloro-1-propanone*

45,3 g de chlorure d'aluminium sont agités à température ambiante dans 80 ml de $CH_2Cl_2$. En maintenant une agitation vigoureuse, on verse lentement une solution du chlorure de l'acide β-propionique (38,09 g, 28,7 ml, 0,3 mol) dans 20 ml de $CH_2Cl_2$. Le complexe $CH_2Cl_2$-$AlCl_3$-chlorure d'acide se forme rapidement et la solution devient rouge sombre. On introduit alors goutte à goutte la solution de bromo benzène (47,1 g, 31,6 ml, 0,3 mol) dans 20 ml de $CH_2Cl_2$. La solution est alors agitée 15 heures à température ambiante. Le mélange est hydrolysé sur 190 g de glace additionnés de 7,6 ml d'acide acétique concentré. La phase organique est lavée jusqu'à neutralisation et le solvant est évaporé sous pression réduite. On obtient une huile rouge sombre dont on extrait à chaud à l'éther de pétrole le produit du titre sous forme d'un solide jaunâtre.

Point de fusion : 60-61°C

*Stade B : 5-Bromo-1-indanone*

Un mélange de 448 g de $AlCl_3$ (335,98 mmol) et de 112 g de NaCl est porté à 180°C dans un réacteur. Le mélange est maintenu sous agitation pendant que l'on introduit lentement à la spatule le composé obtenu au stade A (44,77 g, 180,9 mmol). La température est maintenue à 180-220°C. La réaction se poursuit durant 30 minutes. Après hydrolyse sur 4,5 kg de glace en présence de 135 ml d'acide acétique, on obtient un précipité marron sombre que l'on filtre, lave à l'eau et sèche sous vide. Le produit du titre est isolé par recristallisation dans le méthanol.
Point de fusion : 126-127°C

**Préparation 2 : 1-Oxo-5-indanecarbonitrile**

Un mélange de 5 g du composé obtenu dans la Préparation 1 (23 mmol) et de 2,65 g de CuCN dans 6 ml de DMF est porté à reflux sous argon. Après 15 heures à 120°C sous agitation, le mélange est additionné à une solution de 11,2 g de $FeCl_3$ dans 35 ml d'eau et 6ml d'acide chlorhydrique concentré. Ce mélange est maintenu à 60-71°C pour 15 minutes. Le mélange est extrait avec 3 x 20 ml $NaHCO_3$ 10 %, séché sur $Na_2SO_4$ et concentré sous vide pour conduire au produit du titre sous la forme d'un solide légèrement jaune.
Point de fusion : 129-130°C

**Préparation 3 : 5-(Méthylthio)-1-indanone**

1,27 g de $CH_3SNa$ (18,13 mmol ; 1,2 éq) sont placés à 0°C dans 30 ml de DMF. Le composé obtenu dans la Préparation 1 (3,19 g ; 15,11 mmol) est introduit et le mélange est agité à température ambiante pendant 3 heures. Le mélange réactionnel est ensuite versé sur 150 ml d'eau et extrait par AcOEt. La phase organique est séchée sur $Na_2SO_4$ et concentrée sous vide pour conduire au produit du titre sous la forme d'un produit cristallin brunâtre.
Point de fusion : 99-102°C

**Préparation 4 : 5-Hydroxy-1-indanone**

5 g de 5-méthoxy-1-indanone (30,9 mmol) sont additionnés sous argon à une suspension de 10,31 g de chlorure d'aluminium dans 150 ml de toluène anhydre. La suspension vigoureusement agitée est portée au reflux. Après 30 minutes de réaction, on laisse le mélange revenir à température ambiante et on ajoute 30 g de glace. La phase organique est séparée. La phase aqueuse est lavée avec 2 x 30 ml d'acétate d'éthyle. Les phases organiques sont réunies, lavées avec 4 x 50 ml d'eau, séchées sur $Na_2SO_4$ et concentrées sous vide pour conduire au produit du titre sous la forme d'un solide légèrement brun.
Point de fusion : 183°C

**Préparation 5 : 5-Ethoxy-1-indanone**

3 g du composé obtenu dans la Préparation 4 (20,2 mmol), 5 ml d'iodure d'éthyle et 8,4 g de carbonate de potassium dans 200 ml d'acétone sont portés à reflux sous agitation. Après trois heures de réaction on filtre la suspension, on lave le précipité à l'acétone. On élimine l'acétone sous pression réduite. Le résidu solide est repris dans 25 ml de chloroforme, lavé avec 2 x 10 ml d'eau, préséché avec une solution saturée de chlorure de sodium filtrée sur $Na_2SO_4$, et concentré sous vide. On obtient le produit du titre sous la forme d'un solide légèrement orange.
Point de fusion : 82-83°C

**Préparation 6 : 5-Chloro-1-indanone**

On procède comme dans la Préparation 1 à partir du chloro-benzène.

**Préparation 7 : 5-(Diméthylamino)-1-indanone**

*Stade A :* N-*(2,3-Dihydro-1*H-*indèn-5-yl)acétamide*

A 50 g de 5-aminoindane on additionne goutte à goutte sous agitation un mélange de 70 ml d'anhydride acétique et de 15 g d'acétate de sodium. Après la fin de la réaction exothermique, on chauffe la solution à 100°C une heure. La solution est ensuite versée sur 500 g de glace ; on observe la formation d'un précipité qui est recueilli par

filtration et est repris dans 400 ml d'acétate d'éthyle. La solution est lavée avec 2 x 250 ml d'eau, 2 x 200 ml d'une solution à 20 % d'hydrogénocarbonate de sodium, séchée sur $Na_2SO_4$ et concentrée sous vide. On obtient le produit du titre sous la forme d'un solide jaune.
Point de fusion : 105-106°C

*Stade B :* N-*(1-Oxo-2,3-dihydro-1*H-*indèn-5-yl)acétamide*

A 62 g du composé obtenu au stade A dans un mélange constitué de 175 ml d'acide acétique et 50 ml d'anhydride acétique on additionne goutte à goutte sous agitation une solution de 50 g de trioxyde de chrome en solution dans un mélange de 35 ml d'eau et 150 ml d'acide acétique, de telle sorte que le mélange réactionnel reste à une température inférieure à 10°C. Après une nuit à température ambiante la solution est versée sur 1 litre d'eau glacée. On observe la formation d'un précipité qui est recueilli par filtration. Le précipité est lavé avec de l'eau jusqu'à la neutralité puis séché au dessicateur pour conduire au produit du titre sous la forme d'un solide jaune.
Point de fusion : 172°C

*Stade C : 5-Amino-1-indanone*

47 g du composé obtenu au stade B en solution dans 700 ml d'acide chlorhydrique 1,5 N sont portés à reflux. Après une heure de réaction le produit de départ étant complètement passé en solution, on laisse la solution revenir à température ambiante. Le mélange réactionnel est versé sur 800 ml d'une solution 2M de soude. On filtre le précipité, qui est lavé jusqu'à la neutralité avec de l'eau pour conduire au produit du titre sous la forme d'un solide jaune.
Point de fusion : 184°C

*Stade D : 5-(Diméthylamino)-1-indanone*

5 g du composé obtenu au stade C (20,2 mmol), 8,1 ml d'iodure de méthyle et 7,2 g de carbonate de sodium dans 30 ml d'acétone sont portés à reflux sous agitation. Après une nuit le solvant est éliminé sous vide. On obtient un solide qui est repris dans un mélange constitué de 100 ml d'acétate d'éthyle et de 50 ml d'eau. La phase organique est séparée, lavée avec 4 x 50 ml d'eau, puis 50 ml d'une solution de chlorure de sodium saturé, séchée sur $Na_2SO_4$, et concentrée sous vide. On obtient un produit cristallin légèrement orange qui est purifié par chromatographie sur alumine neutre.
Point de fusion : 116°C

**EXEMPLE 1 : 2-{[3-(Acétylthio)-2-(5-bromo-2,3-dihydro-1*H*-indèn-1-yl)propanoyl] amino}-3-(1*H*-indol-3-yl) propanoate de méthyle**

*Stade A : 5-Bromo-1-indanol*

Le composé obtenu dans la Préparation 1 (72,99 mmol) est suspendu dans 380 ml de MeOH et le borohydrure de sodium (145,97 mmol, 5,54 g) est additionné par portions à température ambiante. Dès que la réaction très exothermique a débuté, la température est controlée par un bain de glace (T < 40°C). Le mélange réactionnel est ensuite agité 4 heures à température ambiante puis est additionné de 60 ml d'eau et concentré sous pression réduite. L'huile obtenue est additionnée de 100 ml d'eau et extraite par 1 x 200 ml d'AcOEt. La phase organique est séparée, lavée par NaCl saturée, séchée sur $Na_2SO_4$ puis concentrée sous pression réduite pour conduire au composé du titre sous la forme d'une huile qui cristallise.
Point de fusion : 79-80°C

*Stade B : 1,5-Dibromoindane*

Le composé obtenu au stade A (70,19 mmol) est solubilisé dans 420 ml de $CHCl_3$ et le bromotriméthylsilane (13,90 ml, 105,28 mmol, 1,5 éq) est ajouté à température ambiante. Le mélange réactionnel est agité la nuit à température ambiante. Le solvant et l'excès de bromotriméthylsilane sont éliminés par évaporation sous pression réduite. L'huile est reprise dans 150 ml de $CHCl_3$, lavée par 2 x 80 ml d'eau, 3 x 100 ml NaCl saturée et séchée sur $Na_2SO_4$. La phase organique est ensuite concentrée sous pression réduite pour conduire au composé du titre sous forme d'une huile marron.

*Stade C : 2-(5-Bromo-2,3-dihydro-1*H*-indèn-1-yl)-2-(diéthoxyphosphoryl)acétate d'éthyle*

Le triéthylphosphonoacétate (15,47 g, 13,63 ml, 69,06 mmol) est placé à 0°C sous argon en solution dans 345 ml de DMF dégazé puis NaH 60 % (3,04 g, 75,97 mmol, 1,1 éq) est additionné par petites quantités. Le mélange réactionnel est agité 30 minutes à température ambiante puis le composé obtenu au stade B (69,06 mmol) est ajouté en une fois. Le mélange est agité la nuit à température ambiante. Le solvant est évaporé sous pression réduite puis le résidu est repris dans 150 ml d'eau et extrait par 2 x 200 ml AcOEt. La phase organique est lavée à l'eau (2 x 100 ml), NaCl saturée (2 x 150 ml), séchée sur $Na_2SO_4$ puis concentrée sous pression réduite pour conduire au composé du titre sous la forme d'une huile.

*Stade D : 2-(5-Bromo-2,3-dihydro-1*H*-indèn-1-yl)acrylate d'éthyle*

Le composé obtenu au stade C (76,18 mmol), le paraformaldéhyde (4,60 g, 152,36 mmol, 2 éq), et $K_2CO_3$ (20,98 g, 2 éq) sont portés à reflux dans 350 ml de THF pendant 15 heures. Le mélange est concentré aux trois quarts, 100 ml d'eau sont ajoutés et le mélange est extrait à l'éther. La phase organique est lavée à l'eau, NaCl saturée, séchée sur $Na_2SO_4$ puis concentrée sous pression réduite pour conduire au composé du titre sous la forme d'une huile.

*Stade E : Acide 2-(5-bromo-2,3-dihydro-1*H*-indèn-1-yl)acrylique*

L'ester obtenu au stade D (76,18 mmol) et 57,1 ml de soude 2N (114,27 mmol, 1,5 éq) sont agités dans 240 ml d'acétone à température ambiante pendant 24 heures. Les solvants sont évaporés et le résidu est repris dans l'eau. La phase aqueuse est extraite à l'éther puis acidifiée par HCl 3N et enfin extraite à l'éther. La phase organique est lavée à l'eau, NaCl saturée, séchée sur $Na_2SO_4$ puis concentrée sous pression réduite pour conduire à l'acide du titre.

*Stade F : Acide 3-(acétylthio)-2-(5-bromo-2,3-dihydro-1*H*-indèn-1-yl)propanoïque*

Le composé éthylénique obtenu au stade E (48,83 mmol) est dissous dans 100 ml de $CHCl_3$ et l'acide thioacétique (170,89 mmol, 3,5 éq) est ajouté. Le mélange est agité à reflux pendant 16 heures. Le solvant et l'excès d'acide thioacétique sont évaporés sous pression réduite pour conduire au composé du titre sous la forme de 4 stéréoisomères séparables en deux couples d'énantiomères :

- par chromatographie HPLC :
  HPLC Kromasil C18 5µ 100A, 250 x 4,6 mm, $CH_3CN$-$H_2O$(0,05 % TFA)60-40
  $R_t$ (2S-3S/2R-3R) = 11,44 min
  $R_t$ (2S-3R/2R-3S) = 12,05 min
  *Masse ESI : 343-345 ($MH^+$)*
- par dédoublement avec une amine chirale :
  8,36 g (24,37 mmol) du mélange obtenu sont solubilisés dans 50 ml $Et_2O$ puis la R(+)-(α)-méthylbenzylamine (1,05 éq., 25,59 mmol, 3,30 ml) est ajoutée. Le mélange est placé 7 jours dans une chambre froide à 4°C. Le précipité est collecté suspendu dans l'éther et HCl 2N est ajouté jusqu'à pH 1. La phase organique est lavée à l'eau, puis séchée sur $Na_2SO_4$. On obtient l'isomère (2S-3R).

*Stade G : 2-{[3-(Acétylthio)-2-(5-bromo-2,3-dihydro-1*H*-indèn-1-yl)propanoyl] amino}-3-(1*H*-indol-3-yl)propanoate de méthyle*

*Procédé général applicable au mélange des 4 diastéréoisomères ou aux couples d'énantiomères :*

Le composé obtenu au stade F (mélange ou couples d'énantiomères) (100 mg) est dissous dans 2 ml d'un mélange THF-$CHCl_3$ 1-1. On additionne à cette solution 1,5 éq d'EDCl, 1,5 éq d'HOBT, 1,5 éq d'$Et_3N$ et 1,5 éq de l'ester méthylique de l'hydrochlorhydrate du (L)tryptophane. Le mélange résultant est agité à température ambiante 4 heures. Les solvants sont éliminés sous pression réduite. Le résidu est repris dans 15 ml d'acétate d'éthyle, la phase organique est lavée avec 3 x 10 ml d'une solution à 10 % d'hydrogénocarbonate de sodium, avec 3 x 10 ml d'une solution à 10 % d'acide citrique, avec 3 x 10 ml d'une solution saturée de chlorure de sodium, séchée sur $Na_2SO_4$ et concentrée sous vide.

Exemple 1a) 2S-3S-4S
Exemple 1b) 2R-3R-4S } Huile, isolés par chromatographie HPLC

Exemple 1c) 2S-3R-4S
Exemple 1d) 2R-3S-4S } Huile, isolés par chromatographie HPLC

**EXEMPLE 2 : 2-{[3-(Acétylthio)-2-(5-cyano-2,3-dihydro-1*H*-indèn-1-yl)propanoyl] amino}-3-(1*H*-indol-3-yl) propanoate de méthyle**

On procède comme dans l'Exemple 1 à partir du composé obtenu dans la Préparation 2. La dernière étape est réalisée sur le mélange des 4 diastéréoisomères.
Huile.

**EXEMPLE 3 : 2-{[3-(Acétylthio)-2-(5-méthylthio-2,3-dihydro-1*H*-indèn-1-yl) propanoyl]amino}-3-(1*H*-indol-3-yl) propanoate de méthyle**

*Stade A : 5-Méthylthio-1-indanol*

On procède comme au stade A de l'Exemple 1 à partir du composé obtenu dans la Préparation 3.

*Stade B : 2-(5-Méthylthio-2,3-dihydro-1*H*-indèn-1-yl)-2-(diéthoxyphosphoryl) acétate d 'éthyle*

A une solution de 20 mmol du composé obtenu au stade A dans 50 ml de THF anhydre à -78°C, on additionne sous agitation et sous argon 22 mmol de bromotriméthyl silane. On laisse remonter la température du mélange à -20°C. Cette solution est additionnée à une solution de l'anion du triéthylphosphonoacétate généré par action de l'hydrure de sodium (20 mmol) sur le triéthylphosphonoacétate (22 mmol). Après une nuit à température ambiante le solvant est éliminé et le résidu purifié par chromatographie sur silice. Le composé du titre est obtenu sous forme d'huile.

*Stade C : 2-{[3-(Acétylthio)-2-(5-méthylthio-2,3-dihydro-1*H*-indèn-1-yl) propanoyl]amino}-3-(1*H*-indol-3-yl)propanoate de méthyle*

On procède comme aux stades D, E, F et G de l'Exemple 1. La dernière étape est réalisée sur le mélange des 4 diastéréoisomères.
Huile.

**EXEMPLE 4 : 2-{[3-(Acétylthio)-2-(5-méthoxy-2,3-dihydro-1*H*-indèn-1-yl) propanoyl]amino}-3-(1*H*-indol-3-yl) propanoate de méthyle**

On procède comme dans l'Exemple 3 à partir de la 5-méthoxy-1-indanone. La dernière étape est réalisée sur le mélange des 4 diastéréoisomères.
Huile.

**EXEMPLE 5 : 2-{[3-(Acétylthio)-2-(4-méthoxy-2,3-dihydro-1*H*-indèn-1-yl) propanoyl]amino}-3-(1*H*-indol-3-yl) propanoate de méthyle**

On procède comme dans l'Exemple 1 à partir de la 4-méthoxy-1-indanone. La dernière étape est réalisée sur le mélange des 4 diastéréoisomères.
Huile.

**EXEMPLE 6 : 2-{[3-(Acétylthio)-2-(6-méthoxy-2,3-dihydro-1*H*-indèn-1-yl) poopanoyl]amino}-3-(1*H*-indol-3-yl) propanoate de méthyle**

On procède comme dans l'Exemple 3 à partir de la 6-méthoxy-1-indanone. La dernière étape est réalisée sur le mélange des 4 diastéréoisomères.

Huile.

**EXEMPLE 7 : 2-{[3-(Acétylthio)-2-(5-hydroxy-2,3-dihydro-1*H*-indèn-1-yl) propanoyl]amino}-3-(1*H*-indol-3-yl) propanoate de méthyle**

*Stade A : Acide 3-(acétylthio)-2-(5-hydroxy-2,3-dihydro-1*H*-indèn-1-yl)propanoïque*

Le composé obtenu au stade F de l'Exemple 1 (mélange des 4 diastéréoisomères) (0,4 g, 1,4 mmol) est dissous dans 3 ml de $CH_2Cl_2$ et la solution obtenue est refroidie à 0°C. On additionne au mélange 1,3 ml d'une solution 1M de tribromure de bore dans $CH_2Cl_2$. Après agitation 15 minutes à température ambiante le mélange est porté à 40°C pour 30 minutes. La solution est ensuite hydrolysée par 6 ml d'eau, puis acidifiée avec 2 ml HCl 1N. La solution est extraite avec 2 x 15 ml d'éther. Les phases organiques sont réunies, lavées deux fois avec 20 ml d'eau puis préséchées avec 15 ml d'une solution saturée de chlorure de sodium, et séchées sur $Na_2SO_4$ puis concentrées sous vide. On obtient une huile qui est purifiée par HPLC semi-préparative.

*Stade B : 2-{[3-(Acétylthio)-2-(5-hydroxy-2,3-dihydro-1*H*-indèn-1-yl) propanoyl]amino}-3-(1*H*-indol-3-yl)propanoate de méthyle*

On procède comme au stade G de l'Exemple 1. La dernière étape est réalisée sur le mélange des 4 diastéréoisomères.
Huile.

**EXEMPLE 8 : 2-{[3-(Acétylthio)-2-(5-éthoxy-2,3-dihydro-1*H*-indèn-1-yl) propanoyl]amino}-3-(1*H*-indol-3-yl) propanoate de méthyle**

On procède comme dans l'Exemple 3 à partir du composé obtenu dans la Préparation 5. La dernière étape est réalisée sur le mélange des 4 diastéréoisomères.
Huile.

**EXEMPLE 9 : 2-{[3-(Acétylthio)-2-(5-chloro-2,3-dihydro-1*H*-indèn-1-yl) propanoyl]amino}-3-(1*H*-indol-3-yl) propanoate de méthyle**

On procède comme dans l'Exemple 1 à partir du composé obtenu dans la Préparation 6. La dernière étape est réalisée sur le mélange des 4 diastéréoisomères.
Huile.

**EXEMPLE 10 : 2-{[3-(Acétylthio)-2-(5-diméthylamino-2,3-dihydro-1*H*-indèn-1-yl) propanoyl]amino}-3-(1*H*-indol-3-yl)propanoate de méthyle**

On procède comme dans l'Exemple 3 à partir du composé obtenu dans la Préparation 7. La dernière étape est réalisée sur le mélange des 4 diastéréoisomères.
Huile.

**EXEMPLE 11 : 2-{[3-(Acétylthio)-2-(2,3-dihydro-1*H*-indèn-1-yl)propanoyl]amino}-3-(1*H*-indol-3-yl)propanoate de méthyle**

On procède comme dans l'Exemple 1 à partir de l'indanone. La dernière étape est réalisée sur le mélange des 4 diastéréoisomères.
Huile.

**EXEMPLE 12 : 2-{[3-(Acétylthio)-2-(1,2,3,4-tétrahydro-1-naphtalényl)propanoyl] amino}-3-(1*H*-indol-3-yl) propanoate de méthyle**

On procède comme dans l'Exemple 1 à partir de la 3,4-dihydro-1(2*H*)-naphtalènone. La dernière étape est réalisée sur le mélange des 4 diastéréoisomères.
Huile.

**EXEMPLE 13 : *N*-[2-(5-Bromo-2,3-dihydro-1*H*-indèn-1-yl)-3-mercaptopropanoyl] tryptophane**

Le composé obtenu dans l'Exemple 1 est dissous dans 2 ml de méthanol dégazé. Après 10 minutes d'agitation à température ambiante on additionne à la solution 6 éq d'une solution de soude 1M dégazée. La solution obtenue est agitée à température ambiante sous argon. L'avancement de la réaction est suivi par HPLC. En fin de réaction on acidifie la solution jusqu'à pH = 1 avec de l'acide chlorhydrique IN. Le méthanol est éliminé sous vide, on ajoute au résidu 10 ml d'eau et on extrait avec 10 ml de chloroforme. La phase organique est séchée sur $Na_2SO_4$ et concentrée sous vide.

| | |
|---|---|
| Exemple 13a) : 2S-3S-4S | $[\alpha]^{15}_D$ = +8,80 |
| | Point de fusion (décomposition) = 163-165°C |
| Exemple 13b) : 2R-3R-4S | $[\alpha]^{15}_D$ = -27,20 |
| | Point de fusion (décomposition) = 135-136°C |
| Exemple 13c) : 2S-3R-4S | $[\alpha]^{15}_D$ = +20,80 |
| | Point de fusion (décomposition) = 126-128°C |
| Exemple 13d) : 2R-3S-4S | $[\alpha]^{15}_D$ = -66,80 |
| | Point de fusion (décomposition) = 130-132°C |

Les Exemples 14 à 24 sont obtenus en procédant comme dans l'Exemple 13 à partir des composés appropriés.

**EXEMPLE 14 : *N*-[2-(5-Cyano-2,3-dihydro-1*H*-indèn-1-yl)-3-mercaptopropanoyl] tryptophane**

Produit de départ : Exemple 2
Solide.
*Masse ESI : 434 ($MH^+$)*

**EXEMPLE 15 : *N*-[2-(5-Méthylthio-2,3-dihydro-1*H*-indèn-1-yl)-3-mercaptopropanoyl]tryptophane**

Produit de départ : Exemple 3
Solide.
*Masse ESI : 455 ($MH^+$)*

**EXEMPLE 16 : *N*-[2-(5-Méthoxy-2,3-dihydro-1*H*-indèn-1-yl)-3-mercaptopropanoyl] tryptophane**

Produit de départ : Exemple 4
Solide.
*Masse ESI : 439 ($MH^+$)*

**EXEMPLE 17 : *N*-[2-(4-Méthoxy-2,3-dihydro-1*H*-indèn-1-yl)-3-mercaptopropanoyl] tryptophane**

Produit de départ : Exemple 5
Solide.
*Masse ESI : 439 ($MH^+$)*

**EXEMPLE 18 : *N*-[2-(6-Méthoxy-2,3-dihydro-1*H*-indèn-1-yl)-3-mercaptopropanoyl] tryptophane**

Produit de départ : Exemple 6
Solide.
*Masse ESI : 439 ($MH^+$)*

**EXEMPLE 19 : *N*-[2-(5-Hydroxy-2,3-dihydro-1*H*-indèn-1-yl)-3-mercaptopropanoyl] tryptophane**

Produit de départ : Exemple 7
Solide.
*Masse ESI : 425 ($MH^+$)*

**EXEMPLE 20 : *N*-[2-(5-Ethoxy-2,3-dihydro-1*H*-indèn-1-yl)-3-mercaptopropanoyl] tryptophane**

Produit de départ : Exemple 8
Solide.
*Masse ESI : 453 ($MH^+$)*

**EXEMPLE 21 : *N*-[2-(5-Chloro-2,3-dihydro-1*H*-indèn-1-yl)-3-mercaptopropanoyl] tryptophane**

Produit de départ : Exemple 9
Solide.
*Masse ESI : 443-445 ($MH^+$)*

**EXEMPLE 22 : *N*-[2-(5-Diméthylamino-2,3-dihydro-1*H*-indèn-1-yl)-3-mercaptopropanoyl]tryptophane**

Produit de départ : Exemple 10
Solide.
*Masse ESI : 452 ($MH^+$)*

**EXEMPLE 23 : *N*-[2-(2,3-Dihydro-1*H*-indèn-1-yl)-3-mercaptopropanoyl] tryptophane**

Produit de départ : Exemple 11
Solide.
*Masse ESI : 409 ($MH^+$)*

**EXEMPLE 24 : *N*-[2-(1,2,3,4-Tétrahydro-1-naphtalényl)-3-mercaptopropanoyl] tryptophane**

Produit de départ : Exemple 12
Solide.
*Masse ESI : 423 ($MH^+$)*

**EXEMPLE 25 : 2-{[3-(Acétylthio)-2-(5-bromo-2,3-dihydro-1*H*-indèn-1-yl)propanoyl] amino}-3-(2-quinolyl)propanoate de méthyle**

Le composé obtenu au stade F de l'Exemple 1 (mélange de 4 diastéréoisomères) (100 mg) est dissous dans 2 ml d'un mélange THF-$CHCl_3$ 1-1. On additionne à cette solution 1,5 éq d'EDCI, 1,5 éq d'HOBT, 1,5 éq d'$Et_3N$ et 1,5 éq de (S)2-amino-3-(2-quinolyl)propanoate de méthyle. Le mélange résultant est agité à température ambiante 4 heures. Les solvants sont éliminés sous pression réduite. Le résidu est repris dans 15 ml d'acétate d'éthyle, la phase organique est lavée, avec 3 x 10 ml d'une solution à 10 % d'hydrogénocarbonate de sodium, avec 3 x 10 ml d'une solution à 10 % d'acide citrique, avec 3 x 10 ml d'une solution saturée de chlorure de sodium, séchée sur $Na_2SO_4$ et concentrée sous vide. Le composé du titre est obtenu après chromatographie sur HPLC.
Huile.

**EXEMPLE 26 : 2-{[3-(Acétylthio)-2-(5-bromo-2,3-dihydro-1*H*-indèn-1-yl)propanoyl] amino}-3-(1-méthyl-1*H*-indol-3-yl)propanoate de méthyle**

On procède comme dans l'Exemple 25 en remplaçant le (S)2-amino-3-(2-quinolyl) propanoate de méthyle par le (S)2-amino-3-(1-méthyl-1*H*-indol-3-yl)propanoate de méthyle.
Huile.

**EXEMPLE 27 : 2-{[3-(Acétylthio)-2-(5-bromo-2,3-dihydro-1*H*-indèn-1-yl)propanoyl] amino}-3-(1*H*-pyrrolo[2,3-*b*] pyridin-3-yl)propanoate de méthyle**

On procède comme dans l'Exemple 25 à partir du diastéréoisomère (2R-3S) purifié du composé obtenu au stade F de l'Exemple 1 et en remplaçant le (S)2-amino-3-(2-quinolyl) propanoate de méthyle par le 2-amino-3-(1*H*-pyrrolo[2,3-b]pyridin-3-yl)propanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05 % TFA) 40-60.

Exemple 27a) 2R-3S-4S : $R_t$ = 9,71 min - Huile

Exemple 27b) 2R-3S-4R : $R_t$ = 12,47 min - Huile

**<u>EXEMPLE 28</u> : 2-{[3-(Acétylthio)-2-(5-bromo-2,3-dihydro-1*H*-indèn-1-yl)propanoyl] amino}-3-(1-benzothièn-3-yl)propanoate de méthyle**

On procède comme dans l'Exemple 25 en remplaçant le (S)2-amino-3-(2-quinolyl) propanoate de méthyle par le (S)2-amino-3-(1-benzothièn-3-yl)propanoate de méthyle. Huile.

**<u>EXEMPLE 29</u> : 2-{[3-(Acétylthio)-2-(5-bromo-2,3-dihydro-1*H*-indèn-1-yl)propanoyl] amino}-3-(1*H*-imidazol-4-yl) propanoate** de méthyle

On procède comme dans l'Exemple 25 en remplaçant le (S)2-amino-3-(2-quinolyl) propanoate de méthyle par le (S)2-amino-3-(1*H*-imidazol-4-yl)propanoate de méthyle.

**<u>EXEMPLE 30</u> : 2-{[3-(Acétylthio)-2-(5-bromo-2,3-dihydro-1*H*-indèn-1-yl)propanoyl] amino}-3-(3-pyridinyl) propanoate de méthyle**

On procède comme dans l'Exemple 25 en remplaçant le (S)2-amino-3-(2-quinolyl) propanoate de méthyle par le (S)2-amino-3-(3-pyridinyl)propanoate de méthyle.

**<u>EXEMPLE 31</u> : N-[2-(5-Bromo-2,3-dihydro-1*H*-indèn-1-yl)-3-mercaptopropanoyl]-3-(2-quinolyl)alanine**

Le composé obtenu dans l'Exemple 25 est dissous dans 2 ml de méthanol dégazé. Après 10 minutes d'agitation à température ambiante on additionne à la solution 6 éq d'une solution de soude 1M dégazée. La solution obtenue est agitée à température ambiante sous argon. L'avancement de la réaction est suivi par HPLC. En fin de réaction on acidifie la solution jusqu'à pH = 1 avec de l'acide chlorhydrique 1N. Le méthanol est éliminé sous vide, on ajoute au résidu 10 ml d'eau et on extrait avec 10 ml de chloroforme. La phase organique est séparée, séchée sur $Na_2SO_4$, concentrée sous vide, et purifiée par chromatographie sur HPLC.
Solide.
*Masse ESI = 499-501 ($MH^+$)*

Les Exemples 32 à 36 sont obtenus en procédant comme dans l'Exemple 31 à partir du substrat approprié.

**<u>EXEMPLE 32</u> : *N*-[2-(5-Bromo-2,3-dihydro-1*H*-indèn-1-yl)-3-mercaptopropanoyl] -1-méthyltryptophane**

<u>Produit de départ</u> : Exemple 26
Solide.
*Masse ESI : 501-503 ($MH^+$)*

**<u>EXEMPLE 33</u> : *N*-[2-(5-Bromo-2,3-dihydro-1*H*-indèn-1-yl)-3-mercaptopropanoyl] -3-(1*H*-pyrrolo[2,3-*b*]pyridin-3-yl)alanine**

<u>Produit de départ</u> : Exemple 27a) ou Exemple 27b)
Solide.
*Masse ESI : 488-490 ($MH^+$)*

Exemple 33a) 2S-3R-4S : $R_t$ = 4,99 min
Exemple 33b) 2S-3R-4R : $R_t$ = 5,49 min

**<u>EXEMPLE 34</u> : 3-(1-Benzothièn-3-yl)-*N*-[2-(5-bromo-2,3-dihydro-1*H*-indèn-1-yl)-3-mercaptopropanoyl]alanine**

<u>Produit de départ</u> : Exemple 28
Solide.
*Masse ESI : 500-502 ($MH^+$)*

**<u>EXEMPLE 35</u> : *N*-[2-(5-Bromo-2,3-dihydro-1*H*-indèn-1-yl)-3-mercaptopropanoyl] histidine**

<u>Produit de départ</u> : Exemple 29
Solide.

*Masse ESI : 438-440 ($MH^+$)*

**<u>EXEMPLE 36</u> : *N*-[2-(5-Bromo-2,3-dihydro-1*H*-indèn-1-yl)-3-mercaptopropanoyl] -3-(3-pyridinyl)alanine**

<u>Produit de départ</u> : Exemple 30
Solide.
*Masse ESI : 448-450 ($MH^+$)*

**<u>EXEMPLE 37</u> : 2-{[3-(Acétylthio)-2-(5-bromo-2,3-dihydro-1*H*-indèn-1-yl)propanoyl] amino}-3-(1-trityl-1*H*-imidazol-4-yl)propanoate de méthyle**

Le composé obtenu au stade F de l'Exemple 1 (300 mg) est dissous dans 8 ml d'un mélange THF-$CHCl_3$ 1-1. On additionne à cette solution 1,5 éq d'EDCI, 1,5 éq d'HOBT, 1,5 éq d'$Et_3N$ et 1,5 éq de (S)2-amino-3-(1-trityl-1*H*-imidazol-4-yl)propanoate de méthyle. Le mélange résultant est agité à température ambiante 4 heures. Les solvants sont éliminés sous pression réduite. Le résidu est repris dans 15 ml d'acétate d'éthyle, la phase organique est lavée, avec 3 x 20 ml d'une solution à 10 % d'hydrogénocarbonnate de sodium, avec 3 x 20 ml d'une solution à 10 % d'acide citrique, avec 3 x 20 ml d'une solution saturée de chlorure de sodium, séchée sur $Na_2SO_4$, concentrée sous vide, et purifiée par chromatographie HPLC.
Huile.

**<u>EXEMPLE 38</u> : *N*-[2-(5-Bromo-2,3-dihydro-1*H*-indèn-1-yl)-3-mercaptopropanoyl] -1-tritylhistidine**

Le composé obtenu dans l'Exemple 37 est dissous dans 5 ml de méthanol dégazé. Après 10 minutes d'agitation à température ambiante on additionne à la solution 4 éq d'une solution de soude 1M dégazée. La solution obtenue est agitée à température ambiante sous argon pendant 5 heures. L'avancement de la réaction est suivi par HPLC. En fin de réaction on acidifie la solution jusqu'à pH = 1 avec de l'acide chlorhydrique 1N. Le méthanol est éliminé sous vide, on ajoute au résidu 10 ml d'eau et on extrait avec 10 ml de chloroforme. La phase organique est séparée, séchée sur $Na_2SO_4$, concentrée sous vide et purifiée par chromatographie HPLC.
Solide.

*<u>Remarque</u> : le composé de l'Exemple 35 peut être obtenu à partir du composé de l'Exempte 38 selon le protocole suivant :*

Le composé obtenu dans l'Exemple 38 est dissous dans 10 mL d'un mélange 95/2,5/2,5 TFA/$H_2O$/Ethanedithiol. Après 30 minutes d'agitation à température ambiante sous argon, les solvants sont éliminés sous vide. Le produit est purifié par HPLC.

**<u>EXEMPLE 39</u> : 2-{[3-(Acétylthio)-2-(5-bromo-2,3-dihydro-1*H*-indèn-1-yl)propanoyl] amino}-3-(7-méthoxy-1*H*-indol-3-yl)propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 2-amino-3-(7-méthoxy-1*H*-indol-3-yl)propanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05% TFA) 70-30.

Exemple 39a) (1$^{er}$ diastéréoisomère) : $R_t$ = 11,72 min - Huile
Exemple 39b) (2$^{ème}$ diastéréoisomère) : $R_t$ = 12,28 min - Huile

**<u>EXEMPLE 40</u> : 2-{[3-(Acétylthio)-2-(5-bromo-2,3-dihydro-1*H*-indèn-1-yl)propanoyl] amino}-3-(5-hydroxy-1*H*-indol-3-yl)propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 2-amino-3-(5-hydroxy-1*H*-indol-3-yl)propanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05% TFA) 60-40.

Exemple 40a) (1$^{er}$ diastéréoisomère) : $R_t$ = 9,20 min - Huile
Exemple 40b) (2$^{ème}$ diastéréoisomère) : $R_t$ = 9,29 min - Huile

**EXEMPLE 41 : 2-{[3-(Acétylthio)-2-(5-bromo-2,3-dihydro-1*H*-indèn-1-yl)propanoyl] amino}-3-(5-méthoxy-1*H*-indol-3-yl)propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 2-amino-3-(5-méthoxy-1*H*-indol-3-yl)propanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05% TFA) 70-30.

Exemple 41a) (1er diastéréoisomère) : $R_t$ = 9,54 min - Huile
Exemple 41b) (2ème diastéréoisomère) : $R_t$ = 10,11 min - Huile

**EXEMPLE 42 : 2-{[3-(Acétylthio)-2-(5-bromo-2,3-dihydro-1*H*-indèn-1-yl)propanoyl] amino}-3-(5-bromo-1*H*-indol-3-yl)propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 2-amino-3-(5-bromo-1*H*-indol-3-yl)propanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05% TFA) 70-30.

Exemple 42a) (1er diastéréoisomère) : $R_t$ = 16,36 min - Huile
Exemple 42b) (2ème diastéréoisomère) : $R_t$ = 16,83 min - Huile

**EXEMPLE 43 : 2-{[3-(Acétylthio)-2-(5-bromo-2,3-dihydro-1*H*-indèn-1-yl)propanoyl] amino}-3-(5-méthyl-1*H*-indol-3-yl)propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 2-amino-3-(5-méthyl-1*H*-indol-3-yl)propanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05% TFA) 70-30.

Exemple 43a) (1er diastéréoisomère) : $R_t$ = 14,10 min - Huile
Exemple 43b) (2ème diastéréoisomère) : $R_t$ = 14,53 min - Huile

**EXEMPLE 44 : 2-{[3-(Acétylthio)-2-(5-bromo-2,3-dihydro-1*H*-indèn-1-yl)propanoyl] amino}-3-(6-méthyl-1*H*-indol-3-yl)propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 2-amino-3-(6-méthyl-1*H*-indol-3-yl)propanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05% TFA) 70-30.

Exemple 44a) (1er diastéréoisomère) : $R_t$ = 14,00 min - Huile
Exemple 44b) (2ème diastéréoisomère) : $R_t$ = 14,54 min - Huile

**EXEMPLE 45 : 2-{[3-(Acétylthio)-2-(5-bromo-2,3-dihydro-1*H*-indèn-1-yl)propanoyl] amino}-3-(6-fluoro-1*H*-indol-3-yl)propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 2-amino-3-(6-fluoro-1*H*-indol-3-yl)propanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05% TFA) 60-40.

Exemple 45a) (1er diastéréoisomère) : $R_t$ = 11,09 min - Huile
Exemple 45b) (2ème diastéréoisomère) : $R_t$ = 11,90 min - Huile

**EXEMPLE 46 : 2-{[3-(Acétylthio)-2-(5-bromo-2,3-dihydro-1*H*-indèn-1-yl)propanoyl] amino}-3-(5-fluoro-1*H*-indol-3-yl)propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 2-amino-3-(5-fluoro-1*H*-indol-3-yl)propanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05% TFA) 70-30.

Exemple 46a) (1er diastéréoisomère) : $R_t$ = 11,13 min - Huile
Exemple 46b) (2ème diastéréoisomère) : $R_t$ = 11,77 min - Huile

**EXEMPLE 47 : 2-{[3-(Acétylthio)-2-(5-bromo-2,3-dihydro-1*H*-indèn-1-yl)propanoyl] amino}-3-(1*H*-indazol-3-yl) propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 2-amino-3-(1*H*-indazol-3-yl)propanoate de méthyle (mélange racémique).
$R_t$ mélange (HPLC Kromasil C18 5µ 100A, 250 x 4,6 mm, $CH_3CN$-$H_2O$ (0,05% TFA) 70-30) = 7,87 min Huile

**EXEMPLE 48 : 2-{[3-(Acétylthio)-2-(5-bromo-2,3-dihydro-1*H*-indèn-1-yl)propanoyl] amino}-3-(1*H*-pyrrolo[2,3-*c*] pyridin-3-yl)propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 2-amino-3-(1*H*-pyrrolo[2,3-*c*]pyridin-3-yl)propanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05% TFA) 50-50.

Exemple 48a) (1er diastéréoisomère) : $R_t$ = 4,70 min - Huile
Exemple 48b) (2ème diastéréoisomère) : $R_t$ = 5,62 min - Huile

**EXEMPLE 49 : 2-{[3-(Acétylthio)-2-(5-bromo-2,3-dihydro-1*H*-indèn-1-yl)propanoyl] amino}-3-(9-acridinyl) propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 3-(9-acridinyl)-2-aminopropanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05% TFA) 50-50.

Exemple 49a) (1er diastéréoisomère) : $R_t$ = 6,48 min - Huile
Exemple 49b) (2ème diastéréoisomère) : $R_t$ = 7,00 min - Huile

**EXEMPLE 50 : 2-{[3-(Acétylthio)-2-(5-bromo-2,3-dihydro-1*H*-indèn-1-yl)propanoyl] amino}-3-(1*H*-pyrrolo[3,2-*h*] quinolin-3-yl)propanoate de méthyle**

On procède comme dans l'Exemple 25 à partir du diastéréoisomère (2S-3R) purifié du composé obtenu au stade F de l'Exemple 1 et en remplaçant le (S)2-amino-3-(2-quinolyl)propanoate de méthyle par le 2-amino-3-(1*H*-pyrrolo[3,2-*h*]quinolin-3-yl)propanoate de méthyle (mélange racémique). Les deux diastéréoisomères obtenus sont séparés par chromatographie HPLC : HPLC Kromasil C18 5µ 100A, 250 x 4,6 mm, $CH_3CN$-$H_2O$ (0,05% TFA) 70-30.

Exemple 50a) (1er diastéréoisomère) : $R_t$ = 3,30 min - Huile
Exemple 50b) (2ème diastéréoisomère) : $R_t$ = 3,87 min - Huile

**EXEMPLE 51 : 2-{[3-(Acétylthio)-2-(5-méthoxy-2,3-dihydro-1*H*-indèn-1-yl) propanoyl]amino}-3-(5-hydroxy-1*H*-indol-3-yl)propanoate de méthyle**

On procède comme dans l'Exemple 1 à partir de la 5-méthoxy-1-indanone, et en condensant au stade G l'ester méthylique du chlorhydrate du 5-hydroxytryptophane sur le diastéréoisomère (2S-3R) purifié du composé obtenu au stade F. Les deux diastéréoisomères obtenus sont séparés par chromatographie HPLC : HPLC Kromasil C18 5µ

100A, 250 x 4,6 mm, $CH_3CN-H_2O$ (0,05% TFA) 60-40.

Exemple 51a) (1er diastéréoisomère) : $R_t$ = 5,80 min - Huile
Exemple 51b) (2ème diastéréoisomère) : $R_t$ = 6,10 min - Huile

**EXEMPLE 52 : *N*-[2-(5-Bromo-2,3-dihydro-1*H*-indèn-1-yl)-3-mercaptopropanoyl]-7-méthoxytryptophane**

On procède comme dans l'Exemple 13 à partir du composé obtenu dans l'Exemple 39 (mélange). Les deux diastéréoisomères obtenus sont séparés par chromatographie HPLC : HPLC Kromasil C18 5µ 100A, 250 x 4,6 mm, $CH_3CN-H_2O$ (0,05% TFA) 70-30.

Exemple 52a) (1er diastéréoisomère) : $R_t$ = 6,57 min - Solide
Exemple 52b) (2ème diastéréoisomère) : $R_t$ = 6,16 min - Solide

*Masse ESI : 517-519 ($MH^+$)*

**EXEMPLE 53 : *N*-[2-(5-Bromo-2,3-dihydro-1*H*-indèn-1-yl)-3-mercaptopropanoyl]-5-hydroxytryptophane**

On procède comme dans l'Exemple 13 à partir du composé obtenu dans l'Exemple 40a) :

Exemple 53a) (2S-3R-4S) : $R_t$ = 3,91 min

(HPLC Kromasil C18 5µ 100A, 250 x 4,6 mm, $CH_3CN-H_2O$ (0,05% TFA) 70-30)
Solide - *Masse ESI : 503-505 ($MH^+$)*

**EXEMPLE 54 : *N*-[2-(5-Bromo-2,3-dihydro-1*H*-indèn-1-yl)-3-mercaptopropanoyl]-5-méthoxytryptophane**

On procède comme dans l'Exemple 13 à partir du composé obtenu dans l'Exemple 41 (mélange). Les deux diastéréoisomères obtenus sont séparés par chromatographie HPLC : HPLC Kromasil C18 5µ 100A, 250 x 4,6 mm, $CH_3CN-H_2O$ (0,05% TFA) 70-30.

Exemple 54a) (2S-3R-4S): $R_t$ = 5,55 min - Solide - *Masse ESI : 517-519 ($MH^+$)*
Exemple 54b) (2S-3R-4R): R, = 5,77 min - Solide - *Masse ESI : 517-519 ($MH^+$)*

**EXEMPLE 55 : 5-Bromo-*N*-[2-(5-bromo-2,3-dihydro-1*H*-indèn-1-yl)-3-mercaptopropanoyl]tryptophane**

On procède comme dans l'Exemple 13 à partir du composé obtenu dans l'Exemple 42 (mélange). Les deux diastéréoisomères obtenus sont séparés par chromatographie HPLC : HPLC Kromasil C18 5µ 100A, 250 x 4,6mm, $CH_3CN-H_2O$ (0,05% TFA) 70-30.

Exemple 55a) (1er diastéréoisomère) : $R_t$ = 7,94 min - Solide *Masse ESI : 565-567-569 ($MH^+$)*
Exemple 55b) (2ème diastéréoisomère) : $R_t$ = 8,63 min - Solide - *Masse ESI : 565-567-569 ($MH^+$)*

**EXEMPLE 56 : *N*-[2-(5-Bromo-2,3-dihydro-1*H*-indèn-1-yl)-3-mercaptopropanoyl]-5-méthyltryptophane**

On procède comme dans l'Exemple 13 à partir du composé obtenu dans l'Exemple 43 (mélange). Les deux diastéréoisomères obtenus sont séparés par chromatographie HPLC : HPLC Kromasil C18 5µ 100A, 250 x 4,6mm, $CH_3CN-H_2O$ (0,05% TFA) 70-30.

Exemple 56a) (1er diastéréoisomère) : $R_t$ = 6,94 min -Solide - *Masse ESI : 501-503 ($MH^+$)*
Exemple 56b) (2ème diastéréoisomère) : $R_t$ = 7,42 min - Solide - *Masse ESI : 501-503 ($MH^+$)*

**EXEMPLE 57 : *N*-[2-(5-Bromo-2,3-dihydro-1*H*-indèn-1-yl)-3-mercaptopropanoyl]-6-méthyltryptophane**

On procède comme dans l'Exemple 13 à partir du composé obtenu dans l'Exemple 44 (mélange). Les deux diastéréoisomères obtenus sont séparés par chromatographie HPLC : HPLC Kromasil C18 5µ 100A, 250 x 4,6mm, $CH_3CN-H_2O$ (0,05% TFA) 70-30.

Exemple 57a) (1er diastéréoisomère) : $R_t$ = 7,13 min - Solide - *Masse ESI : 501-503 (MH+)*
Exemple 57b) (2ème diastéréoisomère) : $R_t$ = 7,67 min - Solide - *Masse ESI : 501-503 (MH+)*

**EXEMPLE 58 : *N*-[2-(5-Bromo-2,3-dihydro-1*H*-indèn-1-yl)-3-mercaptopropanoyl]-6-fluorotryptophane**

On procède comme dans l'Exemple 13 à partir du composé obtenu dans l'Exemple 45 (mélange). Les deux diastéréoisomères obtenus sont séparés par chromatographie HPLC : HPLC Kromasil C18 5μ 100A, 250 x 4,6mm, $CH_3CN$-$H_2O$ (0,05% TFA) 70-30.

Exemple 58a) (1er diastéréoisomère) : $R_t$ = 6,10 min - Solide - *Masse ESI : 505-507 (MH+)*
Exemple 58b) (2ème diastéréoisomère) : $R_t$ = 6,40 min - Solide - *Masse ESI : 505-507 (MH+)*

**EXEMPLE 59 : *N*-[2-(5-Bromo-2,3-dihydro-1*H*-indèn-1-yl)-3-mercaptopropanoyl]-5-fluorotryptophane**

On procède comme dans l'Exemple 13 à partir du composé obtenu dans l'Exemple 46 (mélange). Les deux diastéréoisomères obtenus sont séparés par chromatographie HPLC : HPLC Kromasil C18 5μ 100A, 250 x 4,6mm, $CH_3CN$-$H_2O$ (0,05% TFA) 70-30.

Exemple 59a) (2S-3R-4S) : $R_t$ = 6,09 min - Solide - *Masse ESI : 505-507 (MH+)*
Exemple 59b) (2S-3R-4R) : $R_t$ = 5,99 min - Solide - *Masse ESI : 505-507 (MH-)*

**EXEMPLE 60 : *N*-[2-(5-Bromo-2,3-dihydro-1*H*-indèn-1-yl)-3-mercaptopropanoyl]-3-(1*H*-indazol-3-yl)alanine**

On procède comme dans l'Exemple 13 à partir du composé obtenu dans l'Exemple 47 (mélange). Les deux diastéréoisomères obtenus sont séparés par chromatographie HPLC : HPLC Kromasil C18 5μ 100A, 250 x 4,6mm, $CH_3CN$-$H_2O$ (0,05% TFA) 60-40.

Exemple 60a) (2S-3R-4S) : $R_t$ = 7,22 min - Solide - *Masse ESI : 488-490 (MH+)*
Exemple 60b) (2S-3R-4R) : $R_t$ = 7,75 min - Solide - *Masse ESI : 488-490 (MH-)*

**EXEMPLE 61 : *N*-[2-(5-Bromo-2,3-dihydro-1*H*-indèn-1-yl)-3-mercaptopropanoyl]-3-(1*H*-pyrrolo[2,3-c]pyridin-3-yl)alanine**

On procède comme dans l'Exemple 13 à partir du composé obtenu dans l'Exemple 48 (mélange). Les deux diastéréoisomères obtenus sont séparés par chromatographie HPLC : HPLC Kromasil C18 5μ 100A, 250 x 4,6mm, $CH_3CN$-$H_2O$ (0,05% TFA) 50-50.

Exemple 61a) (1er diastéréoisomère) : $R_t$ = 3,35 min - Solide - *Masse ESI : 488-490 (MH+)*
Exemple 61b) (2ème diastéréoisomère) : R, = 3,62 min - Solide - *Masse ESI : 488-490 (MH+)*

**EXEMPLE 62 : 3-(9-Acridinyl)-*N*-[2-(5-bromo-2,3-dihydro-1*H*-indèn-1-yl)-3-mercaptopropanoyl]alanine**

On procède comme dans l'Exemple 13 à partir du composé obtenu dans l'Exemple 49 (mélange).
$R_t$ mélange (HPLC Kromasil C18 5μ 100A, 250 x 4,6mm, $CH_3CN$-$H_2O$ (0,05% TFA) 50-50) = 4,02 min Solide - *Masse ESI : 546-548 (MH+)*

**EXEMPLE 63 : *N*-[2-(5-Bromo-2,3-dihydro-1*H*-indèn-1-yl)-3-mercaptopropanoyl]-3-(1*H*-pyrrolo[3,2-*h*]quinolin-3-yl)alanine**

On procède comme dans l'Exemple 13 à partir du composé obtenu dans l'Exemple 50 (mélange). Les deux diastéréoisomères obtenus sont séparés par chromatographie HPLC : HPLC Kromasil C18 5μ 100A, 250 x 4,6mm, $CH_3CN$-$H_2O$ (0,05% TFA) 60-40.

Exemple 63a) (2S-3R-4S) : $R_t$ = 3,50 min - Solide - *Masse ESI : 538-540 (MH+)*
Exemple 63b) (2S-3R-4R) : $R_t$ = 3,19 min - Solide - *Masse ESI : 538-540 (MH+)*

**EXEMPLE 64 : 5-Hydroxy-*N*-[3-mercapto-2-(5-méthoxy-2,3-dihydro-1*H*-indèn-1-yl)propanoyl]tryptophane**

On procède comme dans l'Exemple 13 à partir du composé obtenu dans l'Exemple 51 (mélange).
$R_t$ mélange (HPLC Kromasil C18 5µ 100A, 250 x 4,6mm, $CH_3CN$-$H_2O$ (0,05% TFA) 60-40) = 4,00 min Solide

**EXEMPLE 65 : 2-{[3-(Acétylthio)-2-(5,6-diméthoxy-2,3-dihydro-1*H*-indèn-1-yl) propanoyl]amino}-3-(5-hydroxy-1*H*-indol-3-yl)propanoate de méthyle**

On procède comme dans l'Exemple 1 à partir de la 5,6-diméthoxy-l-indanone et en condensant au stade G l'ester méthylique du chlorhydrate du 5-hydroxytryptophane sur le composé obtenu au stade F (mélange).
Huile.

**EXEMPLE 66 : *N*-[2-(5,6-Diméthoxy-2,3-dihydro-1*H*-indèn-1-yl)-3-mercaptopropanoyl]-5-hydroxytryptophane**

On procède comme dans l'Exemple 13 à partir du composé obtenu dans l'Exemple 65 (mélange).
$R_t$ mélange (HPLC Kromasil C18 5µ 100A, 250 x 4,6mm, $CH_3CN$-$H_2O$ (0,05% TFA) 60-40) = 5,16 min Solide

**ETUDE PHARMACOLOGIQUE**

**EXEMPLE A : Inhibition de l'endopeptidase neutre**

L'endopeptidase neutre est purifiée à partir du rein de lapin selon le procédé décrit par Aubry et al. (Biochem. Cell Biol., 1987, 65, pp. 1037-1042).
L'activité enzymatique est mesurée en utilisant le substrat fluorescent, Dansyl-Gly-(p-$NO_2$)Phe-β-Ala-(DGNPA), Km = 37 µM (Goudreau N. et al., Anal. Biochem., 1994, 219, pp. 87-95).
Les composés de l'invention montrent une excellente capacité d'inhibition de l'endopeptidase neutre avec des Ki compris entre 2 et 50 nM.
A titre d'Exemples, les composés des Exemples 33 (2S-3R-4S), 13 (2S-3R-4S) et 63 (2S-3R-4S) ont des Ki de 2,91 ± 0,67 nM, 3,28 ± 0,35 nM et 10,2 ± 0,3 nM respectivement.

**EXEMPLE B : Inhibition de l'enzyme de conversion de l'angiotensine I**

L'ACE est purifiée à partir du testicule de rat selon le procédé décrit par Pantaliano et al. (Biochemistry, 1984, 23, pp. 1037-1042).
L'activité enzymatique est mesurée en utilisant le substrat synthétique N-Cbz-Phe-His-Leu, Km = 50 mM (Piquilloud Y. et al., Biochem. Biophys. Acta, 1970, 206, pp. 136-142).
Les composés de l'invention montrent une excellente capacité d'inhibition de l'enzyme de conversion de l'angiotensine I avec des Ki compris entre 2 et 50 nM.
A titre d'Exemples, les composés des Exemples 33 (2S-3R-4S), 13 (2S-3R-4S) et 63 (2S-3R-4S) ont des Ki de 1,32 ± 0,17 nM, 4,09 ± 0,49 nM et 3,7 ± 0,38 nM respectivement.

**EXEMPLE C : Inhibition de l'enzyme de conversion de l'endothéline (ET)**

Le cDNA de l'ECE-lc (forme membranaire de l'enzyme, Biochem. J., 1997, 328, pp. 871-877), a été inséré dans un vecteur d'expression eucaryote pcDNA3 puis transfecté par électroporation et exprimé dans des cellules Cos-7.
Pour la mesure de l'activité ECE, les cellules sont homogénéisées et la fraction membranaire est récupérée. L'enzyme est extraite par solubilisation dans le β-octylglucose (1 %).

*a) Synthèse du substrat*

Le peptide BigET-1 (19-35) a été préparé par synthèse en phase solide en chimie Fmoc et purifié par HPLC semi-préparative. La propionylation du peptide a été effectuée par action du *N*-succinimidyl-[2,3-$^3$H]-propionate sur le peptide BigET-1 (19-35) et le produit radiomarqué a été purifié par HPLC. L'activité spécifique de ce substrat est de 97 Ci/mmole.

*b) Essai enzymatique*

L'ECE-1c (10 µl d'une solution diluée au 1/10) est solubilisée dans 400 µl de Trismaleate 50 mM, pH 6,5, en présence de 250 mM NaCl. La réaction est initiée par addition de 10 µl du substrat radioactif (concentration finale 1 x $10^{-9}$ M). Après incubation, 1 heure à 37°C, la réaction est stoppée par addition de 600 µl d'acétate d'éthyle. Le métabolite est séparé du substrat intact par extraction liquide-liquide. La radioactivité du métabolite est déterminée par scintillation liquide. Les constantes cinétiques du substrat sont : Km = 17,1 ± 0,6 µM et Vmax = 2,98 ± 0,24 nmol/mg. prot./min. Pour déterminer le Ki des inhibiteurs, ceux-ci sont préincubés 10 minutes à 37°C à différentes concentrations (de $10^{-4}$ à $10^{-10}$ M) avant addition du substrat. Les témoins de 0 % et de 100 % de dégradation sont obtenus respectivement par incubation du substrat avec de l'enzyme inactivée et avec de l'enzyme native en absence d'inhibiteurs.

Les composés de l'invention montrent une excellente capacité d'inhibition de l'enzyme de conversion de la big endothéline avec des Ki compris entre 2 et 50 nM.

A titre d'Exemples, les composés des Exemples 33 (2S-3R-4S), 13 (2S-3R-4S) et 63 (2S-3R-4S) ont des Ki de 23,3 ± 3,2 nM, 24,4 ± 1,4 nM et 21,2 ± 2,5 nM respectivement.

**<u>EXEMPLE D</u> : Composition pharmaceutique**

| | |
|---|---|
| 1000 comprimés dosés à 5 mg de *N*-[2-(5-bromo-2,3-dihydro-1*H*-indèn-1-yl)-3-mercaptopropanoyl] tryptophane (2S-3R-4S) (Exemple 13c)) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

**Revendications**

**1.** Composés de formule (I) :

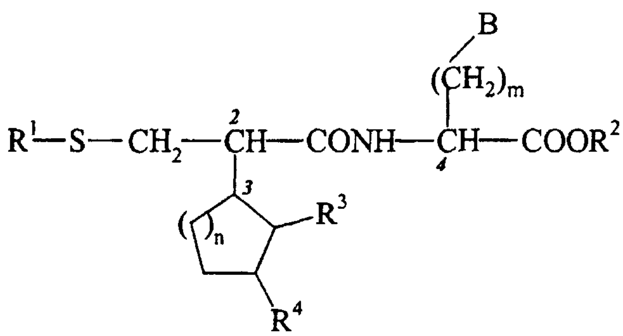

(I)

dans laquelle :

- n représente un nombre entier tel que 0 ≤ n ≤ 3,
- m représente un nombre entier tel que 0 ≤ m ≤ 6,
- $R^3$ et $R^4$ forment ensemble, avec les deux atomes de carbone qui les portent un groupement phényle non substitué ou substitué par 1 à 3 groupements, identiques ou différents, choisis parmi alkyle, alkényle, alkynyle, alkoxy, hydroxy, alkylthio, mercapto, cyano, nitro, amino, alkylamino, dialkylamino, polyhalogénoalkyle, azido, carboxy, alkoxycarbonyle, amido, carbamoyle, formyle, acyle, aryle, hétéroaryle ou atomes d'halogène,
- B représente un groupement hétéroaryle,
- $R^2$ représente un atome d'hydrogène ou un groupement alkyle, alkényle, cycloalkyle, cycloalkylalkyle, acyle, aryle, arylalkyle ou aroyle,

➢ $R^1$ représente un atome d'hydrogène, un groupement acyle, aroyle ou cycloalkylcarbonyle ou un groupement de formule (II) :

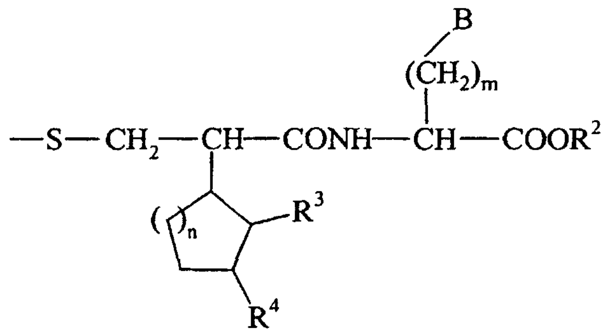

(II)

dans laquelle m, n, $R^2$, $R^3$, $R^4$ et B sont tels que définis précédemment,
étant entendu que :

- par « alkyle » on entend un groupement alkyle à chaîne linéaire ou ramifiée contenant 1 à 6 atomes de carbone,
- par « alkényle » on entend un groupement alkyle contenant 2 à 6 atomes de carbone et une ou plusieurs double-liaisons,
- par « alkynyle » on entend un groupement alkyle contenant 2 à 6 atomes de carbone et une ou plusieurs triple-liaisons,
- par « cycloalkyle » on entend un groupement alkyle cyclique contenant 3 à 8 atomes de carbone,
- par « acyle » on entend un groupement RCO dans lequel R représente un groupement alkyle tel que défini précédemment,
  les groupements « alkyle », « alkényle » et « alkynyle » pouvant être substitués par un ou plusieurs groupements, identiques ou différents, choisis parmi hydroxy, alkoxy, polyhalogénoalkyle, amino ou atomes d'halogène,
  et les groupements « cycloalkyle » et « cycloalkylalkyle » pouvant être substitués sur la partie cyclique par un ou plusieurs groupements, identiques ou différents, choisis parmi hydroxy, alkoxy, polyhalogénoalkyle, amino ou atomes d'halogène,
- par « aryle », on entend un groupement phényle ou naphtyle non substitué ou substitué par ou plusieurs groupements, identiques ou différents, choisis parmi alkyle, alkényle, alkynyle, alkoxy, hydroxy, alkylthio, mercapto, cyano, nitro, amino, alkylamino, dialkylamino, polyhalogénoalkyle, azido, carboxy, alkoxycarbonyle, amido, carbamoyle, formyle, acyle ou atomes d'halogène,
- par « hétéroaryle » on entend tout groupement aromatique mono ou polycyclique contenant 1 à 3 hétéroatomes choisis parmi oxygène, soufre et azote, ces groupements étant non substitués ou substitués par un ou plusieurs groupements, identiques ou différents, choisis parmi alkyle, alkényle, alkynyle, alkoxy, hydroxy, alkylthio, mercapto, cyano, nitro, amino, alkylamino, dialkylamino, polyhalogénoalkyle, azido, carboxy, alkoxycarbonyle, amido, carbamoyle, formyle, acyle ou atomes d'halogène, les groupements polycycliques pouvant être par ailleurs partiellement ou totalement hydrogénés sur un des cycles,
  leurs énantiomères et diastéréoisomères, ainsi que leurs sels d'addition à un acide ou à une base pharmaceutiquement acceptable.

2. Composés de formule (I) selon la revendication 1 pour lesquels $R^1$ représente un atome d'hydrogène, leurs énantiomères et diastéréoisomères, ainsi que leurs sels d'addition à un acide ou à une base pharmaceutiquement acceptable.

3. Composés de formule (I) selon la revendication 1 pour lesquels $R^1$ représente un groupement acyle, leurs énantiomères et diastéréoisomères, ainsi que leurs sels d'addition à un acide ou à une base pharmaceutiquement acceptable.

4. Composés de formule (I) selon la revendication 1 pour lesquels $R^2$ représente un atome d'hydrogène, leurs énantiomères et diastéréoisomères, ainsi que leurs sels d'addition à un acide ou à une base pharmaceutiquement acceptable.

5. Composés de formule (I) selon la revendication 1 pour lesquels $R^2$ représente un groupement alkyle, leurs énantiomères et diastéréoisomères, ainsi que leurs sels d'addition à un acide ou à une base pharmaceutiquement acceptable.

6. Composés de formule (I) selon la revendication 1 pour lesquels $R^3$ et $R^4$ forment ensemble un groupement phényle substitué, leurs énantiomères et diastéréoisomères, ainsi que leurs sels d'addition à un acide ou à une base pharmaceutiquement acceptable.

7. Composés de formule (I) selon la revendication 1 pour lesquels $R^3$ et $R^4$ forment ensemble un groupement phényle substitué par un atome d'halogène ou un groupement méthoxy, leurs énantiomères et diastéréoisomères, ainsi que leurs sels d'addition à un acide ou à une base pharmaceutiquement acceptable.

8. Composés de formule (I) selon la revendication 1 pour lesquels n représente 1, leurs énantiomères et diastéréoisomères, ainsi que leurs sels d'addition à un acide ou à une base pharmaceutiquement acceptable.

9. Composés de formule (I) selon la revendication 1 pour lesquels m représente 1, leurs énantiomères et diastéréoisomères, ainsi que leurs sels d'addition à un acide ou à une base pharmaceutiquement acceptable.

10. Composés de formule (I) selon la revendication 1 pour lesquels B représente un groupement hétéroaryle contenant un groupement NH, leurs énantiomères et diastéréoisomères, ainsi que leurs sels d'addition à un acide ou à une base pharmaceutiquement acceptable.

11. Composés de formule (I) selon la revendication 1 pour lesquels B représente un groupement indolyle, leurs énantiomères et diastéréoisomères, ainsi que leurs sels d'addition à un acide ou à une base pharmaceutiquement acceptable.

12. Composés de formule (I) selon la revendication 1 pour lesquels B représente un groupement pyrrolopyridinyle, leurs énantiomères et diastéréoisomères, ainsi que leurs sels d'addition à un acide ou à une base pharmaceutiquement acceptable.

13. Composés de formule (I) selon la revendication 1 pour lesquels B représente un groupement pyrroloquinolinyle, leurs énantiomères et diastéréoisomères, ainsi que leurs sels d'addition à un acide ou à une base pharmaceutiquement acceptable.

14. Composés de formule (I) selon la revendication 1 de configuration 2S-3R, leurs diastéréoisomères ainsi que leurs sels d'addition à un acide ou à une base pharmaceutiquement acceptable.

15. Composés de formule (I) selon la revendication 1 de configuration 2S-3R-4S ainsi que leurs sels d'addition à un acide ou à une base pharmaceutiquement acceptable.

16. Composé de formule (I) selon la revendication 1 qui est le *N*-[2-(5-bromo-2,3-dihydro-1*H*-indèn-1-yl)-3-mercaptopropanoyl]tryptophane, ses énantiomères et diastéréoisomères, ainsi que ses sels d'addition à un acide ou à une base pharmaceutiquement acceptable.

17. Composé de formule (I) selon la revendication 1 qui est le *N*-[2-(5-bromo-2,3-dihydro-1*H*-indèn-1-yl)-3-mercaptopropanoyl]tryptophane (2S-3R-4S) ainsi que ses sels d'addition à un acide ou à une base pharmaceutiquement acceptable.

18. Composé de formule (I) selon la revendication 1 qui est le *N*-[2-(5-méthylthio-2,3-dihydro-1*H*-indèn-1-yl)-3-mercapto-propanoyl]tryptophane, ses énantiomères et diastéréoisomères, ainsi que ses sels d'addition à un acide ou à une base pharmaceutiquement acceptable.

19. Composé de formule (I) selon la revendication 1 qui est le *N*-[2-(5-méthoxy-2,3-dihydro-1*H*-indèn-1-yl)-3-mercaptopropanoyl]tryptophane, ses énantiomères et diastéréoisomères, ainsi que ses sels d'addition à un acide ou à une base pharmaceutiquement acceptable.

**20.** Composé de formule (I) selon la revendication 1 qui est le *N*-[2-(5-bromo-2,3-dihydro-1*H*-indèn-1-yl)-3-mercapto-propanoyl]-3-(1*H*-pyrrolo[2,3-b]pyridin-3-yl)alanine, ses énantiomères et diastéréoisomères, ainsi que ses sels d'addition à un acide ou à une base pharmaceutiquement acceptable.

**21.** Composé de formule (I) selon la revendication 1 qui est le N-[2-(5-bromo-2,3-dihydro-1*H*-indèn-1-yl)-3-mercapto-propanoyl]-3-(1*H*-pyrrolo[2,3-b]pyridin-3-yl)alanine (2S-3R-4S) ainsi que ses sels d'addition à un acide ou à une base pharmaceutiquement acceptable.

**22.** Composé de formule (I) selon la revendication 1 qui est le N-[2-(5-bromo-2,3-dihydro-1*H*-indèn-1-yl)-3-mercapto-propanoyl]-5-méthoxytryptophane (2S-3R-4S) ainsi que ses sels d'addition à un acide ou à une base pharmaceutiquement acceptable.

**23.** Composé de formule (I) selon la revendication 1 qui est le *N*-[2-(5-bromo-2,3-dihydro-1*H*-indèn-1-yl)-3-mercapto-propanoyl]-5-fluorotryptophane (2S-3R-4S) ainsi que ses sels d'addition à un acide ou à une base pharmaceutiquement acceptable.

**24.** Composé de formule (I) selon la revendication 1 qui est le *N*-[2-(5-bromo-2,3-dihydro-1*H*-indèn-1-yl)-3-mercapto-propanoyl]-3-(1*H*-pyrolo[3,2-h]quinolin-3-yl)alanine (2S-3R-4S) ainsi que ses sels d'addition à un acide ou à une base pharmaceutiquement acceptable.

**25.** Procédé de préparation des composés de formule (I) selon la revendication 1 **caractérisé en ce que** l'on utilise comme produit de départ le composé de formule (III) :

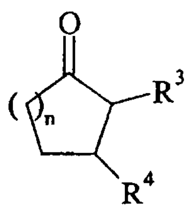

(III)

dans laquelle $R^3$, $R^4$ et n sont tels que définis dans la formule (I),
que l'on soumet à un agent réducteur comme $NaBH_4$ par exemple pour obtenir le composé de formule (IV) :

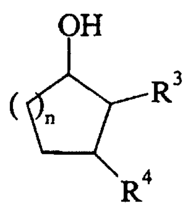

(IV)

dans laquelle n, $R^3$ et $R^4$ sont définis comme précédemment,
que l'on transforme avec un agent d'halogénation comme $Me_3SiBr$ par exemple en dérivé halogéné correspondant de formule (V) :

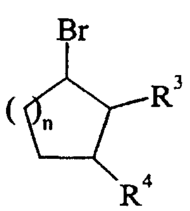

(V)

dans laquelle $R^3$, $R^4$ et n sont tels que définis précédemment,

sur lequel on condense, en milieu basique le 2-(diéthoxyphosphoryl)acétate d'éthyle pour conduire au composé de formule (VI) :

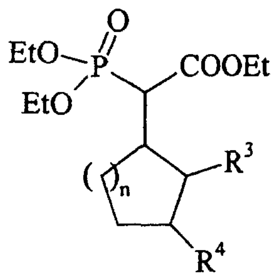

(VI)

dans laquelle $R^3$, $R^4$ et n sont définis comme précédemment,
sur lequel on fait réagir du formaldéhyde en milieu basique pour obtenir le composé de formule (VII) :

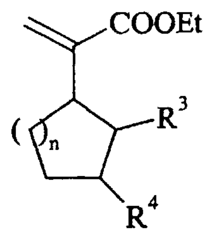

(VII)

dans laquelle $R^3$, $R^4$ et n sont définis comme précédemment,
qui est saponifié en présence de soude pour conduire au composé de formule (VIII) :

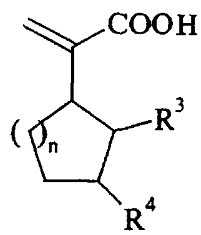

(VIII)

dans laquelle $R^3$, $R^4$ et n sont définis comme précédemment,
sur lequel on condense un composé de formule (IX) :

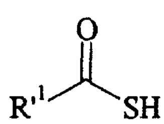

(IX)

dans laquelle $R'^1$ représente un groupement alkyle, aryle ou cycloalkyle,
pour obtenir le composé de formule (X) :

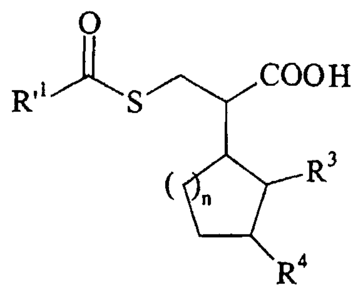

(X)

dans laquelle $R^3$, $R^4$, $R'^1$ et n sont tels que définis précédemment,
sur lequel on condense, en présence d'un agent de couplage comme le 1-(3-diméthylaminopropyl)-3-éthylcarbodiimide par exemple, un composé de formule (XI) :

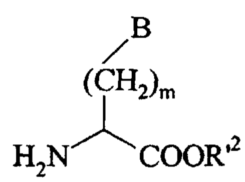

(XI)

dans laquelle B et m sont tels que définis dans la formule (I), et $R'^2$ représente un groupement alkyle, alkényle, cycloalkyle, cycloalkylalkyle, aryle, acyle, arylakyle ou aroyle,
pour conduire au composé de formule (I/a), cas particulier des composés de formule (I) :

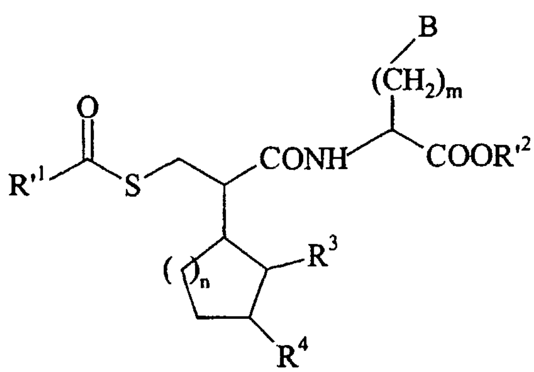

(I/a)

dans laquelle $R'^1$, $R^3$, $R^4$, $R'^2$, m, n et B sont définis de la même façon que précédemment,
qui peut être partiellement ou totalement saponifié en milieu basique pour conduire au composé de formule (I/b), cas particulier des composés de formule (I) :

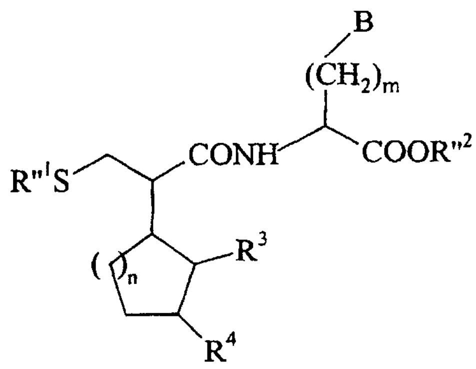

(I/b)

dans laquelle $R^3$, $R^4$, m, n et B sont tels que définis précédemment, R"' représente un groupement $R'^1$ ou un atome d'hydrogène, et $R''^2$ représente un groupement $R'^2$ ou un atome d'hydrogène, étant entendu que l'un au moins des groupements $R''^1$ et $R''^2$ représente un atome d'hydrogène,

composé de formule (I/b) qui peut être placé, lorsque $R''^1$ représente un atome d'hydrogène, en milieu oxydant pour obtenir le composé de formule (I/c), cas particulier des composés de formule (I) :

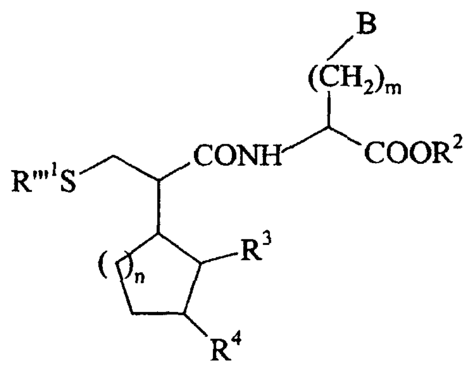

(I/c)

dans laquelle $R^2$, $R^3$, $R^4$, m, n et B sont tels que définis précédemment, et $R'''^1$ représente un groupement de formule (II),

les composés de formule (I/a) à (I/c) formant la totalité des composés de l'invention, et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

**26.** Procédé de préparation selon la revendication 25 des composés de formule (I) selon la revendication 1 **caractérisé en ce que** l'on utilise comme produit de départ le composé de formule (X) tel que défini précédemment dont on sépare par chromatographie les diastéréoisomères, pour conduire aux composés de formule (Xa) et (Xb) :

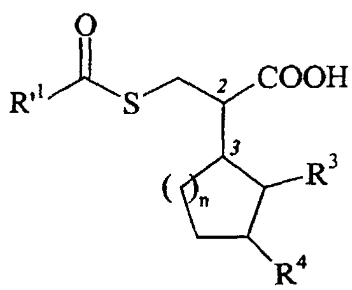

(2R 3R, 2S 3S) (Xa)
(2R 3S, 2S 3R) (Xb)

dans laquelle $R'^1$, $R^3$, $R^4$ et n sont définis comme précédemment,

composé de formule (Xb) dont on peut former un sel avec une amine chirale comme la (R)-(+)-α-méthylbenzylamine par exemple, pour conduire, après dédoublement par recristallisations successives au composé de formule (Xb'):

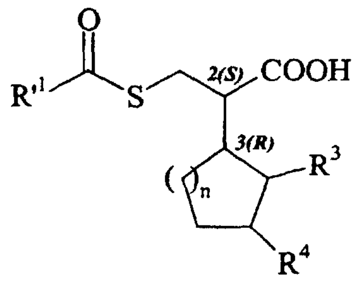

(2S, 3R) (Xb')

dans laquelle $R'^1$, $R^3$, $R^4$ et n sont tels que définis précédemment,
sur lequel on condense, en présence d'un agent de couplage comme le EDCI, un composé de formule (XIa) :

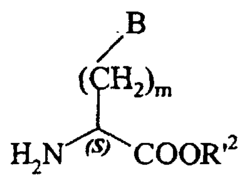

(XIa)

dans laquelle $R'^2$, m et B sont tels que définis précédemment,
pour obtenir le composé de formule (I/a'), cas particulier des composés de formule (I/a) :

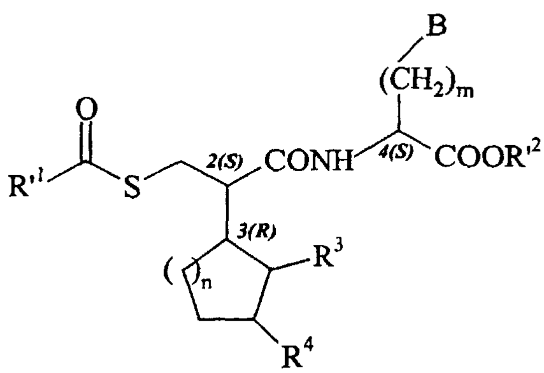

(I/a')

dans laquelle $R'^1$, $R'^2$, $R^3$, $R^4$, m, n et B sont définis de la même façon que précédemment,
les diastéréoisomères (2R, 3S), (2R, 3R) et (2S, 3R) étant obtenus de façon analogue à partir des composés (Xa) ou (Xb) correspondants,
ces composés pouvant par ailleurs être obtenus par condensation du composé de formule (XIa) sur le composé de formule (Xa) ou (Xb) suivie d'une séparation chromatographique.

**27.** Compositions pharmaceutiques contenant comme principe actif au moins un composé de formule (I) selon l'une quelconque des revendications 1 à 24 ou un de ses sels d'addition à un acide ou à une base pharmaceutiquement acceptable, seul ou en combinaison avec un ou plusieurs excipients pharmaceutiquement acceptables.

**28.** Compositions pharmaceutiques selon la revendication 27 utiles pour la fabrication d'un médicament pour le traitement des hypertensions artérielles comprenant les hypertensions artérielles pulmonaires, des ischémies myocardiques, de l'angine de poitrine, des insuffisances cardiaques, des vasculopathies comprenant les vasculopathies diabétiques, de l'athérosclérose et de la resténose angioplastie, des insuffisances rénales aiguës ou chroniques, des maladies cérébrovasculaires comprenant le stroke et les hémorragies sous arachnoïdiennes, des ischémies périphériques, ainsi que de la toxicité à la cyclosporine.

**Patentansprüche**

**1.** Verbindungen der Formel (I):

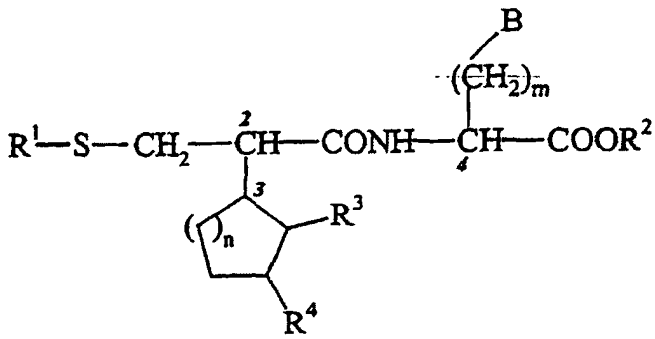

(I)

in der:

- n eine ganze Zahl bedeutet, so daß die Beziehung 0 ≤ n s 3 erfüllt ist,
- m eine ganze Zahl bedeutet, so daß die Beziehung 0 ≤ m ≤ 6 erfüllt ist,
- $R^3$ und $R^4$ gemeinsam mit den beiden sie tragenden Kohlenstoffatomen eine Phenylgruppe bilden, die nichtsubstituiert ist oder durch 1 bis 3 gleichartige oder verschiedene Gruppen ausgewählt aus Alkyl, Alkenyl, Alkinyl, Alkoxy, Hydroxy, Alkylthio, Mercapto, Cyano, Nitro, Amino, Alkylamino, Dialkylamino, Polyhalogenalkyl, Azido, Carboxy, Alkoxycarbonyl, Amido, Carbamoyl, Formyl, Acyl, Aryl, Heteroaryl oder Halogenatomen substituiert ist,
- B eine Heteroarylgruppe bedeutet,
- $R^2$ ein Wasserstoffatom oder eine Alkyl-, Alkenyl-, Cycloalkyl-, Cycloalkylalkyl-, Acyl-, Aryl-, Arylalkyl- oder Aroylgruppe darstellt,
- $R^1$ ein Wasserstoffatom, eine Acyl-, Aroyl- oder Cycloalkylcarbonylgruppe oder eine Gruppe der Formel (II) bedeutet:

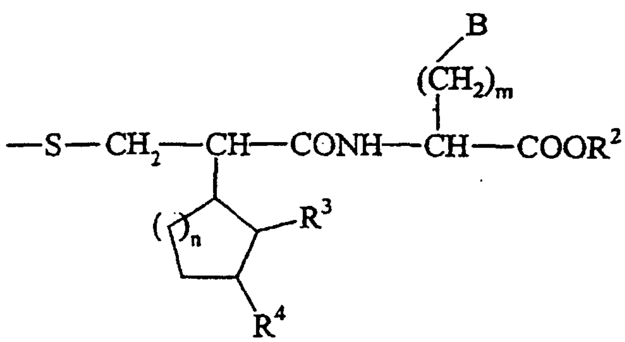

(II)

in der m, n, $R^2$, $R^3$, $R^4$ und B die oben angegebenen Bedeutungen besitzen, wobei es sich versteht, daß man:

- unter «Alkyl» eine Alkylgruppe mit gerader oder verzweigter Kette versteht, die 1 bis 6 Kohlenstoffatome enthält,
- unter «Alkenyl» eine Alkylgruppe versteht, die 2 bis 6 Kohlenstoffatome und eine oder mehrere Doppelbindungen enthält,
- unter «Alkinyl» eine Alkylgruppe versteht, die 2 bis 6 Kohlenstoffatome und eine oder mehrere Dreifachbindungen enthält,

- unter «Cycloalkyl» eine cyclische Alkylgruppe versteht, die 3 bis 8 Kohlenstoffatome enthält,
- unter «Acyl» eine Gruppe RCO versteht, in der R eine Alkylgruppe darstellt, wie sie oben definiert worden ist,

wobei die «Alkyl»-, «Alkenyl»- und «Alkinyl»-gruppen durch eine oder mehrere gleichartige oder verschiedenartige Gruppen ausgewählt aus Hydroxy, Alkoxy, Polyhalogenalkyl, Amino oder Halogenatomen substituiert sein können, und die «Cycloalkyl»- und «Cycloalkylalkyl»-gruppen am cyclischen Teil durch eine oder mehrere gleichartige oder verschiedenartige Gruppen substituiert sein können ausgewählt aus Hydroxy, Alkoxy, Polyhalogenalkyl, Amino oder Halogenatomen.

- unter «Aryl» eine Phenyl- oder Naphthylgruppe versteht, die nichtsubstituiert ist oder durch eine oder mehrere gleichartige oder verschiedenartige Gruppen ausgewählt aus Alkyl, Alkenyl, Alkinyl, Alkoxy, Hydroxy, Alkylthio, Mercapto, Cyano, Nitro, Amino, Alkylamino, Dialkylamino, Polyhalogenalkyl, Azido, Carboxy, Alkoxycarbonyl, Amido, Carbamoyl, Formyl, Acyl und Halogenatomen substituiert ist,
- unter «Heteroaryl» jede aromatische mono- oder polycyclische Gruppe zu verstehen ist, die 1 bis 3 Heteroatome ausgewählt aus Sauerstoff, Schwefel und Stickstoff enthält, wobei diese Gruppen nichtsubstituiert sind oder durch eine oder mehrere gleichartige oder verschiedenartige Gruppen ausgewählt aus Alkyl, Alkenyl, Alkinyl, Alkoxy, Hydroxy, Alkylthio, Mercapto, Cyano, Nitro, Amino, Alkylamino, Dialkylamino, Polyhalogenalkyl, Azido, Carboxy, Alkoxycarbonyl, Amido, Carbamoyl, Formyl, Acyl oder Halogenatomen substituiert sind,

wobei die polycyclischen Gruppen andererseits an den Ringen teilweise oder vollständig hydriert sein können, deren Enantiomere und Diastereoisomere sowie deren Additionssalze mit einer pharmazeutisch annehmbaren Säure oder Base.

**2.** Verbindungen der Formel (I) nach Anspruch 1, worin $R^1$ ein Wasserstoffatom bedeutet, deren Enantiomere und Diastereoisomere sowie deren Additionssalze mit einer pharmazeutisch annehmbaren Säure oder Base.

**3.** Verbindungen der Formel (I) nach Anspruch 1, worin $R^1$ eine Acylgruppe bedeutet, deren Enantiomere und Diastereoisomere sowie deren Additionssalze mit einer pharmazeutisch annehmbaren Säure oder Base.

**4.** Verbindungen der Formel (I) nach Anspruch 1, worin $R^2$ ein Wasserstoffatom bedeutet, deren Enantiomere und Diastereoisomere sowie deren Additionssalze mit einer pharmazeutisch annehmbaren Säure oder Base.

**5.** Verbindungen der Formel (I) nach Anspruch 1, worin $R^2$ eine Alkylgruppe bedeutet, deren Enantiomere und Diastereoisomere sowie deren Additionssalze mit einer pharmazeutisch annehmbaren Säure oder Base.

**6.** Verbindungen der Formel (I) nach Anspruch 1, worin $R^3$ und $R^4$ gemeinsam eine substituierte Phenylgruppe bilden, deren Enantiomere und Diastereoisomere sowie deren Additionssalze mit einer pharmazeutisch annehmbaren Säure oder Base.

**7.** Verbindungen der Formel (I) nach Anspruch 1, worin $R^3$ und $R^4$ gemeinsam eine durch ein Halogenatom oder eine Methoxygruppe substituierte Phenylgruppe bilden, deren Enantiomere und Diastereoisomere sowie deren Additlonssalze mit einer pharmazeutisch annehmbaren Säure oder Base.

**8.** Verbindungen der Formel (I) nach Anspruch 1, worin n 1 bedeutet, deren Enantiomere und Diastereoisomere sowie deren Additionssalze mit einer pharmazeutisch annehmbaren Säure oder Base.

**9.** Verbindungen der Formel (I) nach Anspruch 1, worin m 1 bedeutet, deren Enantiomere und Diastereoisomere sowie deren Additionssalze mit einer pharmazeutisch annehmbaren Säure oder Base.

**10.** Verbindungen der Formel (I) nach Anspruch 1, worin B eine Heteroarylgruppe bedeutet, die eine Gruppe NH enthält, deren Enantiomere und Diastereoisomere sowie deren Additionssalze mit einer pharmazeutisch annehmbaren Säure oder Base.

**11.** Verbindungen der Formel (I) nach Anspruch 1, worin B eine Indolylgruppe bedeutet, deren Enantiomere und Diastereoisomere sowie deren Additionssalze mit einer pharmazeutisch annehmbaren Säure oder Base.

**12.** Verbindungen der Formel (I) nach Anspruch 1, worin B eine Pyrrolopyridinylgruppe bedeutet, deren Enantiomere

und Diastereoisomere sowie deren Additionssalze mit einer pharmazeutisch annehmbaren Säure oder Base.

**13.** Verbindungen der Formel (I) nach Anspruch 1, worin B eine Pyrrolochinolinylgruppe bedeutet, deren Enantiomere und Diastereoisomere sowie deren Additionssalze mit einer pharmazeutisch annehmbaren Säure oder Base.

**14.** Verbindungen der Formel (I) nach Anspruch 1 in der Konfiguration 2S-3R, deren Diastereoisomere sowie deren Additionssalze mit einer pharmazeutisch annehmbaren Säure oder Base.

**15.** Verbindungen der Formel (I) nach Anspruch 1 in der Konfiguration 2S-3R-4S sowie deren Additionssalze mit einer pharmazeutisch annehmbaren Säure oder Base.

**16.** Verbindung der Formel (I) nach Anspruch 1, nämlich *N*-[2-(5-Brom-2,3-dihydro-1*H*-inden-1-yl)-3-mercaptopropanoyl]-tryptophan, dessen Enantiomere und Diastereoisomere sowie dessen Additionssalze mit einer pharmazeutisch annehmbaren Säure oder Base.

**17.** Verbindung der Formel (I) nach Anspruch 1, nämlich *N*-[2-(5-Brom-2,3-dihydro-1*H*-inden-1-yl)-3-mercaptopropanoyl]-tryptophan (2S-3R-4S), sowie dessen Additionssalze mit einer pharmazeutisch annehmbaren Säure oder Base.

**18.** Verbindung der Formel (I) nach Anspruch 1, nämlich *N*-[2-(5-Methylthio-2,3-dihydro-1*H*-inden-1-yl)-3-mercaptopropanoyl]-tryptophan, dessen Enantiomere und Diastereoisomere sowie dessen Additionssalze mit einer pharmazeutisch annehmbaren Säure oder Base.

**19.** Verbindung der Formel (I) nach Anspruch 1, nämlich *N*-[2-(5-Methoxy-2,3-dihydro-1*H*-inden-1-yl)-3-mercaptopropanoyl]-tryptophan, dessen Enantiomere und Diastereoisomere sowie dessen Additionssalze mit einer pharmazeutisch annehmbaren Säure oder Base.

**20.** Verbindung der Formel (I) nach Anspruch 1, nämlich *N*-(2-(5-Brom-2,3-dihydro-1*H*-inden-1-yl)-3-mercaptopropanoyl]-3-(1*H*-pyrrolo[2,3-b]pyridin-3-yl)-alanin, dessen Enantiomere und Diastereoisomere sowie dessen Additionssalze mit einer pharmazeutisch annehmbaren Säure oder Base.

**21.** Verbindung der Formel (I) nach Anspruch 1, nämlich *N*-[2-(5-Brom-2,3-dihydro-1*H*-inden-1-yl)-3-mercaptopropanoyl]-3-(1*H*-pyrrolo[2,3-b]pyridin-3-yl)-alanin (2S-3R-4S), sowie dessen Additionssalze mit einer pharmazeutisch annehmbaren Säure oder Base.

**22.** Verbindung der Formel (I) nach Anspruch 1, nämlich N-[2-(5-Brom-2,3-dihydro-1*H*-inden-1-yl)-3-mercaptopropanoyl]-5-methoxytryptophan (2S-3R-4S), sowie dessen Additionssalze mit einer pharmazeutisch annehmbaren Säure oder Base.

**23.** Verbindung der Formel (I) nach Anspruch 1, nämlich *N*-[2-(5-Brom-2,3-dihydro-1*H*-inden-1-yl)-3-mercaptopropanoyl]-5-fluortryptophan (2S-3R-4S), sowie dessen Additionssalze mit einer pharmazeutisch annehmbaren Säure oder Base.

**24.** Verbndung der Formel (I) nach Anspruch 1, nämlich N-[2-(5-Brom-2,3-dihydro-1*H*-inden-1-yl)-3-mercaptopropanoyl]-3-(1*H*-pyrrolo[3,2-h]chinolin-3-yl)-alanin (2S-3R-4S), sowie dessen Additionssalze mit einer pharmazeutisch annehmbaren Säure oder Base.

**25.** Verfahren zur Herstellung der Verbindungen der Formel (I) nach Anspruch 1, **dadurch gekennzeichnet, daß** als Ausgangsprodukt die Verbindung der Formel (III) verwendet:

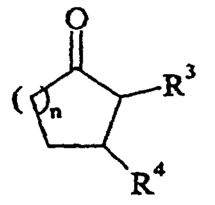

(III)

in der $R^3$, $R^4$ und n die bezüglich der Formel (I) angegebenen Bedeutungen besitzen, welche man mit einem Reduktionsmittel, wie beispielsweise $NaBH_4$, behandelt zur Bildung der Verbindung der Formel (IV):

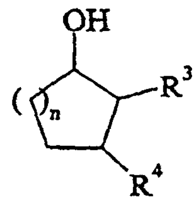

(IV)

in der n, $R^3$ und $R^4$ die oben angegebenen Bedeutungen besitzen, welche man mit einem Halogenierungsmittel, wie beispielsweise $Me_3SiBr$, in das entsprechende Halogenderivat der Formel (V) umwandelt:

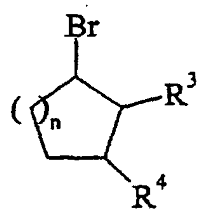

(V)

in der $R^3$, $R^4$ und n die oben angegebenen Bedeutungen besitzen, welches man in basischem Medium mit 2-(Diethoxyphosphoryl)-essigsäureethylester kondensiert zur Bildung der Verbindung der Formel (VI):

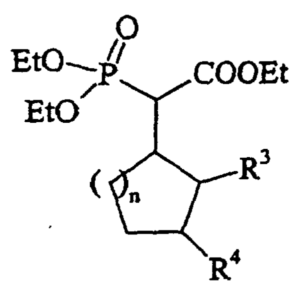

(VI)

in der $R^3$, $R^4$ und n die oben angegebenen Bedeutungen besitzen, welche man mit Formaldehyd in basischem Medium umsetzt zur Bildung der Verbindung der Formel (VII):

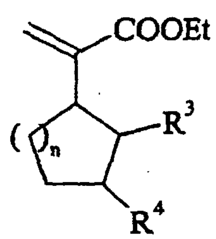

(VII)

in der $R^3$, $R^4$ und n die oben angegebenen Bedeutungen besitzen, welche man in Gegenwart von Natriumhydroxid verseift zur Bildung der Verbindung der Formel (VIII):

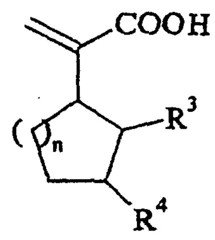

(VIII)

in der $R^3$, $R^4$ und n die oben angegebenen Bedeutungen besitzen, welche man mit einer Verbindung der Formel (IX) kondensiert:

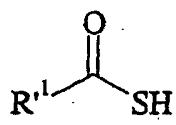

(IX)

in der $R'^1$ eine Alkyl-, Aryl- oder Cycloalkylgruppe bedeutet, zur Bildung der Verbindung der Formel (X):

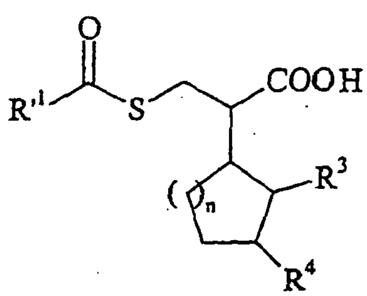

(X)

in der $R^3$, $R^4$, $R'^1$ und n die oben angegebenen Bedeutungen besitzen, welche man in Gegenwart eines Kupplungsmittels, wie beispielsweise 1-(3-Dimethylaminopropyl)-3-ethylcarbodiimid, mit einer Verbindung der Formel (XI) kondensiert:

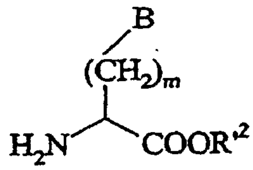

(XI)

in der B und m die bezüglich der Formel (I) angegebenen Bedeutungen besitzen und $R'^2$ eine Alkyl-, Alkenyl-, Cycloalkyl-, Cycloalkylalkyl-, Aryl-, Acyl-, Arylalkyl- oder Aroylgruppe bedeutet, zur Bildung der Verbindung der Formel (I/a), einem Sonderfall der Verbindungen der Formel (I):

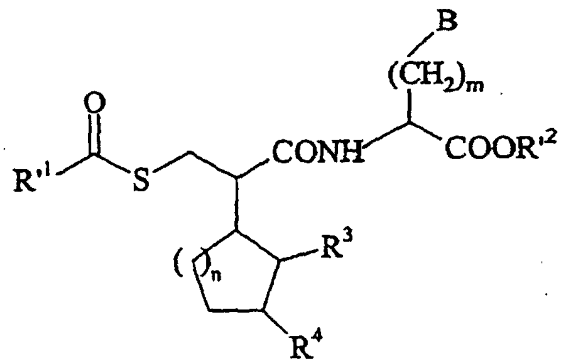

(I/a)

in der R'1, $R^3$, $R^4$, R'2, m, n und B die oben angegebenen Bedeutungen besitzen, welche in basischem Medium teilweise oder vollständig verseift werden kann zur Bildung der Verbindung der Formel (I/b), einem Sonderfall der Verbindungen der Formel (I):

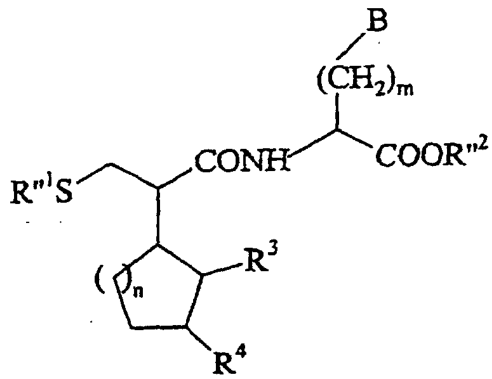

(I/b)

in der $R^3$, $R^4$, m, n und B die oben angegebenen Bedeutungen besitzen, R"1 eine Gruppe R'1 oder ein Wasserstoffatom und R"2 eine Gruppe R'2 oder ein Wasserstoffatom bedeuten, mit der Maßgabe, daß mindestens eine der Gruppen R"1 und R"2 ein Wasserstoffatom darstellt.
welche Verbindung der Formel (I/b) dann, wenn R"1 ein Wasserstoffatom bedeutet, in ein oxidierendes Medium eingebracht werden kann zur Bildung der Verbindung der Formel (I/c), einem Sonderfall der Verbindungen der Formel (I):

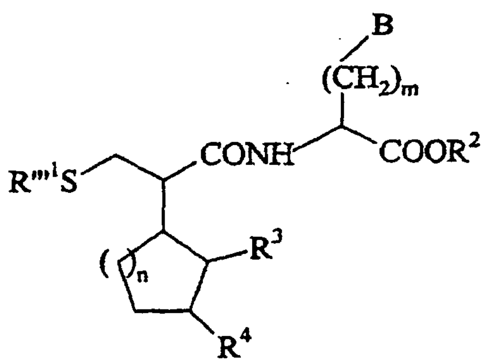

(I/c)

in der $R^2$, $R^3$, $R^4$, m, n und B die oben angegebenen Bedeutungen besitzen und R"'1 eine Gruppe der Formel (II) bedeutet,
welche Verbindungen der Formeln (I/a) bis (I/c), welche die Gesamtheit der erfindungsgemäßen Verbindungen

bilden, mit Hilfe einer klassischen Trennmethode gereinigt werden können, welche gewünschtenfalls in ihre Additionssalze mit einer pharmazeutisch annehmbaren Säure oder Base überführt werden können und welche gegebenenfalls mit Hilfe einer klassischen Trennmethode in ihre Isomeren aufgetrennt werden können.

**26.** Verfahren nach Anspruch 25 zur Herstellung der Verbindungen der Formel (I) gemäß Anspruch 1, **dadurch gekennzeichnet, daß** man als Ausgangsprodukt die Verbindung der Formel (X), wie sie oben definiert worden ist, verwendet, welche man chromatographisch in die Diastereoisomeren auftrennt, so daß man die Verbindungen der Formeln (Xa) und (Xb) erhält:

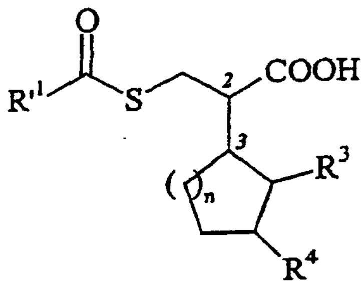

(2R 3R, 2S 3S) (Xa)
(2R 3S, 2S 3R) (Xb)

in der $R'^1$, $R^3$, $R^4$ und n die oben angegebenen Bedeutungen besitzen,
wonach man mit der Verbindung der Formel (Xb) ein Salz mit einem chiralen Amin, wie beispielsweise (R)-(+)-α-Methylbenzylamin, bilden kann, so daß man nach der Trennung durch aufeinanderfolgende Umkristallisationen die Verbindung der Formel (Xb') erhält:

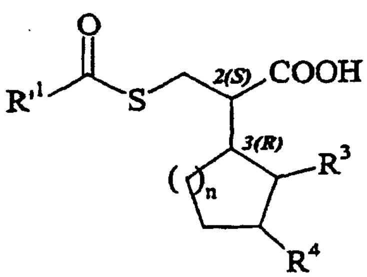

(2S, 3R) (Xb')

in der $R'^1$, $R^3$, $R^4$ und n die oben angegebenen Bedeutungen besitzen,
welche man in Gegenwart eines Kupplungsmittels, wie EDCI, mit einer Verbindung der Formel (XIa) kondensiert:

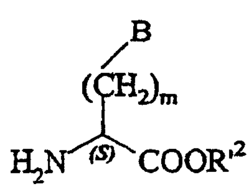

(XIa)

in der $R'^2$, m und B die oben angegebenen Bedeutungen besitzen,
so daß man die Verbindung der Formel (I/a') erhält, einem Sonderfall der Verbindungen der Formel (I/a):

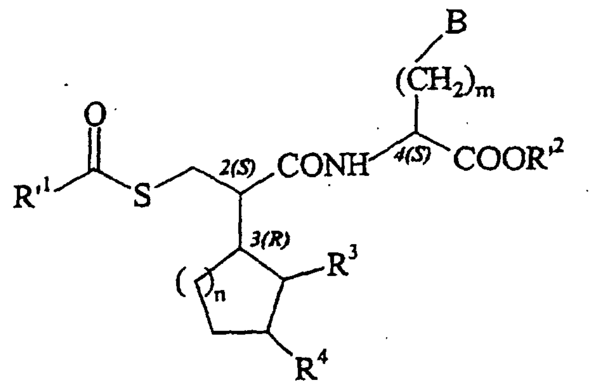

(I/a')

in der $R'^1$, $R'^2$, $R^3$, $R^4$, m, n und B die oben angegebenen Bedeutungen besitzen, wobei man die Diastereoisomeren (2R, 3S), (2R, 3R) und (2S, 3R) in analoger Weise ausgehend von den entsprechenden Verbindungen (Xa) oder (Xb) erhält, welche Verbindungen andererseits auch durch Kondensation der Verbindung der Formel (XIa) mit der Verbindung der Formel (Xa) oder (Xb), gefolgt von einer chromatographischen Trennung erhalten werden können.

**27.** Pharmazeutische Zubereitungen, enthaltend als Wirkstoff mindestens eine Verbindung der Formel (I) nach irgendeinem der Ansprüche 1 bis 24 oder eines ihrer Additionssalze mit einer pharmazeutisch annehmbaren Säure oder Base-, allein oder in Kombination mit einem oder mehreren pharmazeutisch annehmbaren Trägermaterialien.

**28.** Pharmazeutische Zubereitungen nach Anspruch 27, nützlich für die Herstellung eines Arzneimittels zur Behandlung von arteriellen Hypertensionen, welche arterielle Lungenhypertensionen umfassen, Myokardischämien, Angina pectoris, Herzinusffizienzen, Gefäßerkrankungen einschließlich diabetischer Gefäßerkrankungen, der Atherosklerose und der angioplastischen Restenose, akuten oder chronischen Niereninsuffizienzen, Zerebralgefäßerkrankungen einschließlich Schlaganfällen und subarachnoidalen Blutungen, peripheren Ischämien sowie Cyclosporin-Toxizitätserkrankungen.

## Claims

**1.** Compounds of formula (I):

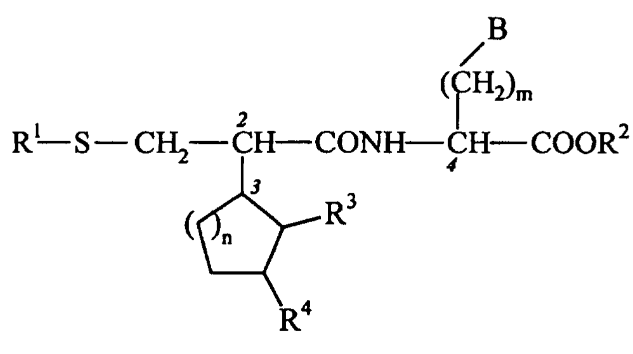

(I)

wherein:

- n represents an integer wherein $0 \leq n \leq 3$,
- m represents an integer wherein $0 \leq m \leq 6$,
- $R^3$ and $R^4$ together form, with the two carbon atoms carrying them, a phenyl group that is unsubstituted or substituted by from 1 to 3 identical or different groups selected from alkyl, alkenyl, alkynyl, alkoxy, hydroxy, alkylthio, mercapto, cyano, nitro, amino, alkylamino, dialkylamino, polyhaloalkyl, azido, carboxy, alkoxycarbonyl, amido, carbamoyl, formyl, acyl, aryl, heteroaryl and halogen atoms,

➢ B represents a heteroaryl group,

➢ $R^2$ represents a hydrogen atom or an alkyl, alkenyl, cycloalkyl, cycloalkylalkyl, acyl, aryl, arylalkyl or aroyl group,

➢ $R^1$ represents a hydrogen atom, an acyl, aroyl or cycloalkylcarbonyl group or a group of formula (II) :

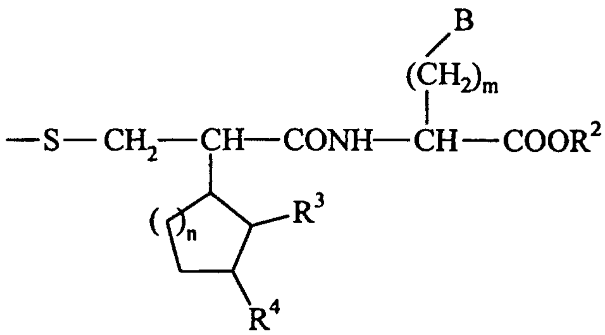

(II)

wherein m, n, $R^2$, $R^3$, $R^4$ and B are as defined hereinbefore,
it being understood that:

- "alkyl" is understood to mean an alkyl group having a linear or branched chain containing from 1 to 6 carbon atoms,
- "alkenyl" is understood to mean an alkyl group containing from 2 to 6 carbon atoms and one or more double bonds,
- "alkynyl" is understood to mean an alkyl group containing from 2 to 6 carbon atoms and one or more triple bonds,
- "cycloalkyl" is understood to mean a cyclic alkyl group containing from 3 to 8 carbon atoms,
- "acyl" is understood to mean an RCO group wherein R represents an alkyl group as defined hereinbefore, it being possible for the groups "alkyl", "alkenyl" and "alkynyl" to be substituted by one or more identical or different groups selected from hydroxy, alkoxy, polyhaloalkyl, amino and halogen atoms, and it being possible for the groups "cycloalkyl" and "cycloalkylalkyl" to be substituted on the cyclic moiety by one or more identical or different groups selected from hydroxy, alkoxy, polyhaloalkyl, amino and halogen atoms,
- "aryl" is understood to mean a phenyl or naphthyl group unsubstituted or substituted by one or more identical or different groups selected from alkyl, alkenyl, alkynyl, alkoxy, hydroxy, alkylthio, mercapto, cyano, nitro, amino, alkylamino, dialkylamino, polyhaloalkyl, azido, carboxy, alkoxycarbonyl, amido, carbamoyl, formyl, acyl and halogen atoms,
- "heteroaryl" is understood to mean any mono- or poly-cyclic aromatic group containing from 1 to 3 hetero atoms selected from oxygen, sulphur and nitrogen, those groups being unsubstituted or substituted by one or more identical or different groups selected from alkyl, alkenyl, alkynyl, alkoxy, hydroxy, alkylthio, mercapto, cyano, nitro, amino, alkylamino, dialkylamino, polyhaloalkyl, azido, carboxy, alkoxycarbonyl, amido, carbamoyl, formyl, acyl and halogen atoms,
  it being possible for the polycyclic groups also to be partially or completely hydrogenated on one of the rings, their enantiomers and diastereoisomers, and addition salts thereof with a pharmaceutically acceptable acid or base.

**2.** Compounds of formula (I) according to claim 1 wherein $R^1$ represents a hydrogen atom, their enantiomers and diastereoisomers, and addition salts thereof with a pharmaceutically acceptable acid or base.

**3.** Compounds of formula (I) according to claim 1 wherein $R^1$ represents an acyl group, their enantiomers and diastereoisomers, and addition salts thereof with a pharmaceutically acceptable acid or base.

**4.** Compounds of formula (I) according to claim 1 wherein $R^2$ represents a hydrogen atom, their enantiomers and diastereoisomers, and addition salts thereof with a pharmaceutically acceptable acid or base.

**5.** Compounds of formula (I) according to claim 1 wherein $R^2$ represents an alkyl group, their enantiomers and diastereoisomers, and addition salts thereof with a pharmaceutically acceptable acid or base.

**6.** Compounds of formula (I) according to claim 1 wherein $R^3$ and $R^4$ together form a substituted phenyl group, their enantiomers and diastereoisomers, and addition salts thereof with a pharmaceutically acceptable acid or base.

**7.** Compounds of formula (I) according to claim 1 wherein $R^3$ and $R^4$ together form a phenyl group substituted by a halogen atom or by a methoxy group, their enantiomers and diastereoisomers, and addition salts thereof with a pharmaceutically acceptable acid or base.

**8.** Compounds of formula (I) according to claim 1 wherein n is 1, their enantiomers and diastereoisomers, and addition salts thereof with a pharmaceutically acceptable acid or base.

**9.** Compounds of formula (I) according to claim 1 wherein m is 1, their enantiomers and diastereoisomers, and addition salts thereof with a pharmaceutically acceptable acid or base.

**10.** Compounds of formula (I) according to claim 1 wherein B represents a heteroaryl group containing an NH group, their enantiomers and diastereoisomers, and addition salts thereof with a pharmaceutically acceptable acid or base.

**11.** Compounds of formula (I) according to claim 1 wherein B represents an indolyl group, their enantiomers and diastereoisomers, and addition salts thereof with a pharmaceutically acceptable acid or base.

**12.** Compounds of formula (I) according to claim 1 wherein B represents a pyrrolopyridyl group, their enantiomers and diastereoisomers, and addition salts thereof with a pharmaceutically acceptable acid or base.

**13.** Compounds of formula (I) according to claim I wherein B represents a pyrroloquinolyl group, their enantiomers and diastereoisomers, and addition salts thereof with a pharmaceutically acceptable acid or base.

**14.** Compounds of formula (I) according to claim 1 of configuration 2S,3R, their diastereoisomers and addition salts thereof with a pharmaceutically acceptable acid or base.

**15.** Compounds of formula (I) according to claim 1 of configuration 2S,3R,4S and addition salts thereof with a pharmaceutically acceptable acid or base.

**16.** Compound of formula (I) according to claim 1 which is *N*-[2-(5-bromo-2,3-dihydro-1*H*-inden-1-yl)-3-mercaptopropanoyl]tryptophan, its enantiomers and diastereoisomers, and addition salts thereof with a pharmaceutically acceptable acid or base.

**17.** Compound of formula (I) according to claim 1 which is *N*-[2-(5-bromo-2,3-dihydro-1*H*-inden-1-yl)-3-mercaptopropanoyl]tryptophan (2S,3R,4S) and addition salts thereof with a pharmaceutically acceptable acid or base.

**18.** Compound of formula (I) according to claim 1 which is *N*-[2-(5-methylthio-2,3-dihydro-1*H*-inden-1-yl)-3-mercaptopropanoyl]tryptophan, its enantiomers and diastereoisomers, and addition salts thereof with a pharmaceutically acceptable acid or base.

**19.** Compound of formula (I) according to claim 1 which is *N*-[2-(5-methoxy-2,3-dihydro-1*H*-inden-1-yl)-3-mercaptopropanoyl]tryptophan, its enantiomers and diastereoisomers, and addition salts thereof with a pharmaceutically acceptable acid or base.

**20.** Compound of formula (I) according to claim 1 which is *N*-[2-(5-bromo-2,3-dihydro-1*H*-inden-1-yl)-3-mercaptopropanoyl]-3-(1*H*-pyrrolo[2,3-b]pyrid-3-yl)alanine, its enantiomers and diastereoisomers, and addition salts thereof with a pharmaceutically acceptable acid or base.

**21.** Compound of formula (I) according to claim 1 which is *N*-[2-(5-bromo-2,3-dihydro-1*H*-inden-1-yl)-3-mercaptopropanoyl]-3-(1*H*-pyrrolo[2,3-b]pyrid-3-yl)alanine (2S,3R,4S) and addition salts thereof with a pharmaceutically acceptable acid or base.

**22.** Compound of formula (I) according to claim 1 which is *N*-[2-(5-bromo-2,3-dihydro-1*H*-inden-1-yl)-3-mercaptopropanoyl]-5-methoxytryptophan (2S,3R,4S) and addition salts thereof with a pharmaceutically acceptable acid or base.

**23.** Compound of formula (I) according to claim 1 which is *N*-[2-(5-bromo-2,3-dihydro-1*H*-inden-1-yl)-3-mercaptopropanoyl]-5-fluorotryptophan (2S,3R,4S) and addition salts thereof with a pharmaceutically acceptable acid or base.

**24.** Compound of formula (I) according to claim 1 which is *N*-[2-(5-bromo-2,3-dihydro-1*H*-inden-1-yl)-3-mercaptopropanoyl]-3-(1*H*-pyrrolo[3,2-h]quinol-3-yl)alanine (2S,3R,4S) and addition salts thereof with a pharmaceutically acceptable acid or base.

**25.** Process for the preparation of compounds of formula (I) according to claim 1, **characterised in that** there is used as starting material a compound of formula (III) :

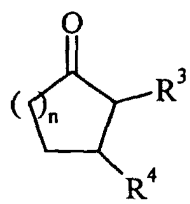

(III)

wherein $R^3$, $R^4$ and n are as defined for formula (I),
which is subjected to the action of a reducing agent, such as $NaBH_4$ for example, to obtain a compound of formula (IV) :

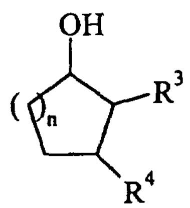

(IV)

wherein n, $R^3$ and $R^4$ are as defined hereinbefore,
which is converted with a halogenating agent, such as $Me_3SiBr$ for example, to a corresponding halogen compound of formula (V) :

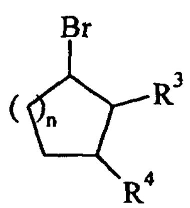

(V)

wherein $R^3$, $R^4$ and n are as defined hereinbefore,
which is condensed, in a basic medium, with ethyl 2-(diethoxyphosphoryl)acetate to yield a compound of formula (VI) :

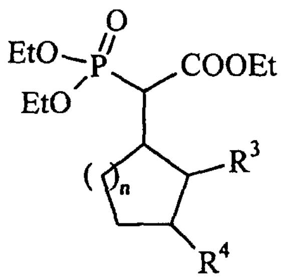

(VI)

wherein $R^3$, $R^4$ and n are as defined hereinbefore,
which is reacted with formaldehyde in a basic medium to obtain a compound of formula (VII):

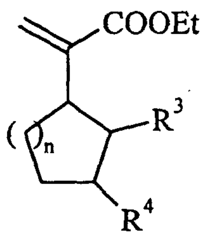

(VII)

wherein $R^3$, $R^4$ and n are as defined hereinbefore,
which is hydrolysed in the presence of sodium hydroxide to yield a compound of formula (VIII) :

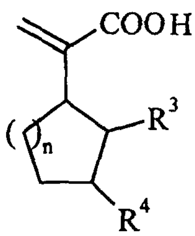

(VIII)

wherein $R^3$, $R^4$ and n are as defined hereinbefore,
which is condensed with a compound of formula (IX) :

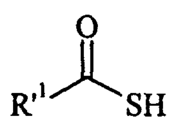

(IX)

wherein $R'^1$ represents an alkyl, aryl or cycloalkyl group,
to obtain a compound of formula (X):

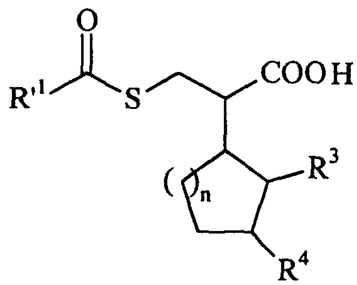

(X)

wherein $R^3$, $R^4$, $R'^1$ and n are as defined hereinbefore,
which is condensed, in the presence of a coupling agent, such as 1-(3-dimethylamino-propyl)-3-ethylcarbodiimide for example, with a compound of formula (XI) :

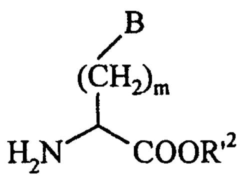

(XI)

wherein B and m are as defined for formula (I), and $R'^2$ represents an alkyl, alkenyl, cycloalkyl, cycloalkylalkyl, aryl, acyl, arylalkyl or aroyl group,
to yield a compound of formula (I/a), a particular case of the compounds of formula (I) :

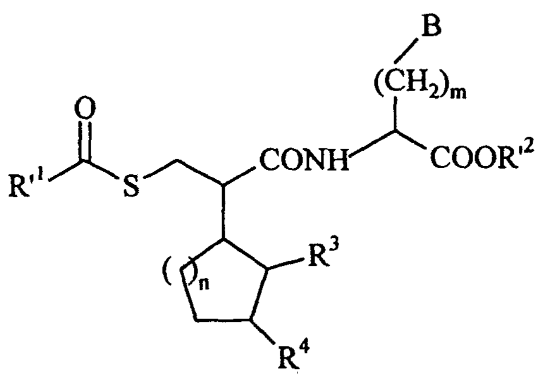

(I/a)

wherein $R'^1$, $R^3$, $R^4$, $R'^2$, m, n and B are as defined hereinbefore,
which may be partially or completely hydrolysed in a basic medium to yield a compound of formula (I/b), a particular case of the compounds of formula (I):

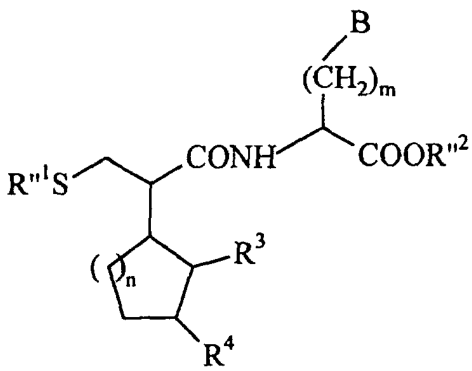

(I/b)

wherein $R^3$, $R^4$, m, n and B are as defined hereinbefore, $R''^1$ represents a group $R'^1$ or a hydrogen atom, and $R''^2$ represents a group $R'^2$ or a hydrogen atom, with the proviso that at least one of the groups $R''^1$ and $R''^2$ represents a hydrogen atom,
which compound of formula (I/b), when $R''^1$ represents a hydrogen atom, may be placed in an oxidising medium to obtain a compound of formula (I/c), a particular case of the compounds of formula (I) :

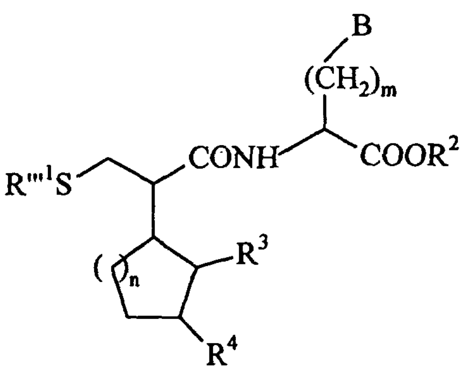

(I/c)

wherein $R^2$, $R^3$, $R^4$, m, n and B are as defined hereinbefore, and $R'''^1$ represents a group of formula (II),
which compounds of formulae (I/a) to (I/c) constitute the totality of the compounds of the invention, and may be purified in accordance with a conventional separation technique, are converted, if desired, into addition salts thereof with a pharmaceutically acceptable acid or base, and are separated, where appropriate, into their isomers in accordance with a conventional separation technique.

**26.** Process according to claim 25 for the preparation of compounds of formula (I) according to claim 1, **characterised in that** there is used as starting material a compound of formula (X) as defined hereinbefore, the diastereoisomers of which are separated by chromatography to yield the compounds of formulae (Xa) and (Xb) :

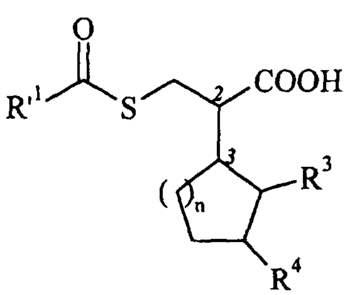

(2R,3R; 2S,3S) (Xa)
(2R,3S; 2S,3R) (Xb)

wherein $R'^1$, $R^3$, $R^4$ and n are as defined hereinbefore,

with which compound of formula (Xb) it is possible to form a salt with a chiral amine, such as (R)-(+)-α-methylbenzylamine for example, to yield, after resolution by successive recrystallisation operations, a compound of formula (Xb') :

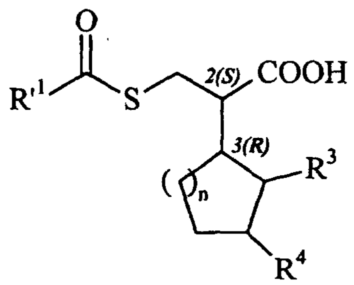

(2S, 3R) (Xb')

wherein $R'^1$, $R^3$, $R^4$ and n are as defined hereinbefore,
which is condensed, in the presence of a coupling agent, such as EDCI, with a compound of formula (XIa) :

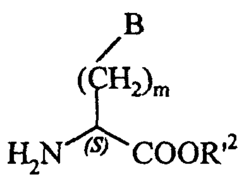

(XIa)

wherein $R'^2$, m and B are as defined hereinbefore,
to obtain a compound of formula (I/a'), a particular case of the compounds of formula (I/a) :

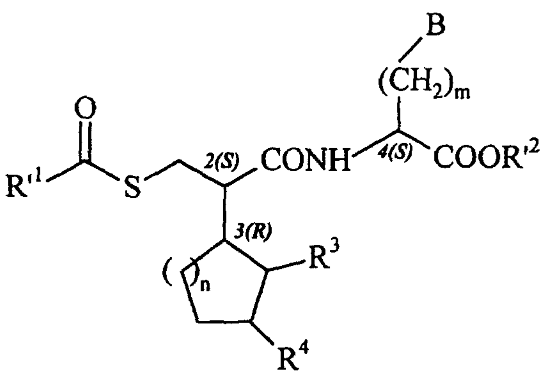

(I/a')

wherein $R'^1$, $R'^2$, $R^3$, $R^4$, m, n and B are as defined hereinbefore,
the diastereoisomers (2R,3S), (2R,3R) and (2S,3R) being obtained analogously starting from the corresponding compounds (Xa) or (Xb),
it also being possible to obtain those compounds by condensing a compound of formula (XIa) with a compound of formula (Xa) or (Xb) followed by separation by chromatography.

**27.** Pharmaceutical compositions comprising as active ingredient at least one compound of formula (I) according to any one of claims 1 to 24 or an addition salt thereof with a pharmaceutically acceptable acid or base, alone or in combination with one or more pharmaceutically acceptable excipients.

**28.** Pharmaceutical compositions according to claim 27 for use in the manufacture of a medicament for the treatment of arterial hypertension including pulmonary arterial hypertension, myocardial ischaemia, angina pectoris, cardiac insufficiency, vasculopathies including diabetic vasculopathies, atherosclerosis and angioplasty restenosis, acute or chronic renal insufficiency, cerebrovascular diseases including stroke and sub-arachnoidal haemorrhage, peripheral ischaemia, and cyclosporin toxicity.